# EUROPÄISCHE PATENTSCHRIFT

(11) **EP 2 092 085 B1**
(45) Veröffentlichungstag und Bekanntmachung des Hinweises auf die Patenterteilung: **09.04.2014**
(21) Anmeldenummer: 07870194.3
(22) Anmeldetag: 01.11.2007
(51) Int. Cl.: C12Q 1/68

(54) **PROGNOSTISCHE MARKER FÜR DIE KLASSIFIZIERUNG DES DREIJÄHRIGEN PROGRESSIONSFREIEN ÜBERLEBENS VON PATIENTEN MIT KOLOREKTALEM KARZINOM BASIEREND AUF EXPRESSIONSPROFILEN VON BIOLOGISCHEN PROBEN**
PROGNOSTIC MARKER FOR CLASSIFYING THE THREE-YEAR PROGRESSION-FREE SURVIVAL OF PATIENTS WITH COLORECTAL CARCINOMA BASED ON EXPRESSION PROFILES OF BIOLOGICAL SAMPLES
MARQUEURS PRONOSTIQUES POUR LA CLASSIFICATION DE LA SURVIE À TROIS ANS SANS PROGRESSION DE PATIENTS AYANT UN CARCINOME COLORECTAL SUR LA BASE DE PROFILS D'EXPRESSION D'ÉCHANTILLONS BIOLOGIQUES

(30) Priorität: 02.11.2006 DE 102006035393
(43) Veröffentlichungstag der Anmeldung: 26.08.2009
(73) Patentinhaber: Signature Diagnostics AG, 14473 Potsdam (DE)
(72) Erfinder: ADAMS, Hans-Peter, 14469 Potsdam (DE); MAYR, Tobias, 10435 Berlin (DE); CLEVERT, Djörk-Arné, 10437 Berlin (DE); HINZMANN, Bernd, 13127 Berlin (DE)
(74) Vertreter: Hoppe, Georg Johannes
(86) Internationale Anmeldenummer: PCT/DE2007/050003
(87) Internationale Veröffentlichungsnummer: WO 2008/061525

(56) Entgegenhaltungen:
- EP-A1- 2 028 492
- WO-A-2004/005923
- WO-A-2004/031774
- WO-A-2005/066371
- WO-A-2007/012811
- WO-A-2007/015947
- WO-A-2007/073220
- WO-A-2007/135174
- WO-A-2008/045133
- WO-A1-2007/142347
- CRONER ROLAND S ET AL: "Microarray versus conventional prediction of lymph node metastasis in colorectal carcinoma" CANCER, AMERICAN CANCER SOCIETY, PHILADELPHIA, PA, US, Bd. 104, Nr. 2, 10. Juni 2005 (2005-06-10), Seiten 395-404, XP009088697 ISSN: 0008-543X
- WANG Y ET AL: "Gene expression profiles and molecular markers to predict recurrence of Dukes' B colon cancer" JOURNAL OF CLINICAL ONCOLOGY, GRUNE AND STRATTON, NEW YORK, NY, US, Bd. 22, Nr. 9, 1. Mai 2004 (2004-05-01), Seiten 1564-1571, XP003014769 ISSN: 0732-183X
- GRABOWSKI P ET AL: "Expression of sialyl-Le(x) antigen defined by MAb AM-3 is an independent prognostic marker in colorectal carcinoma patients" INTERNATIONAL JOURNAL OF CANCER 2000 US, Bd. 88, Nr. 2, 2000, Seiten 281-286, XP002478779 ISSN: 0020-7136
- SCHWANDNER O ET AL: "p21, p27, cyclin D1, and p53 in rectal cancer: Immunohistology with prognostic significance?" INTERNATIONAL JOURNAL OF COLORECTAL DISEASE, Bd. 17, Nr. 1, Januar 2002 (2002-01), Seiten 11-19, XP002478780 ISSN: 0179-1958
- ADACHI YASUSHI ET AL: "Expression of angiomodulin (tumor-derived adhesion factor/mac25) in invading tumor cells correlates with poor prognosis in human colorectal cancer" INTERNATIONAL JOURNAL OF CANCER, Bd. 95, Nr. 4, 20. Juli 2001 (2001-07-20), Seiten 216-222, XP002478781 ISSN: 0020-7136
- MULDER JAN-WILLEM R ET AL: "Colorectal cancer prognosis and expression of exon-v6-containing CD44 proteins" LANCET (NORTH AMERICAN EDITION), Bd. 344, Nr. 8935, 1994, Seiten 1470-1472, XP002478782 ISSN: 0099-5355
- KAMOTO ICHIRO C ET AL: "Seven novel and stable translocations associated with oncogenic gene expression in malignant melanoma" NEOPLASIA (NEW YORK), Bd. 7, Nr. 4, April 2005 (2005-04), Seiten 303-311, XP002478778 ISSN: 1522-8002
- CERESA B P: "Regulation of EGFR endocytic trafficking by rab proteins" HISTOLOGY AND HISTOPATHOLOGY 200607 ES, Bd. 21, Nr. 7-9, Juli 2006 (2006-07), Seiten 987-993, XP009099289 ISSN: 0213-3911

## Beschreibung

Die Erfindung umfasst ein Verfahren zur Vorhersage (Prädiktion) der Progression einer Dickdarmkrebserkrankung (kolorektales Karzinom) innerhalb von drei Jahren bei Patienten, die mit Dickdarmkrebs im UICC Stadium I und II nach dem Stand der Technik diagnostiziert wurden und deren primärer Tumor nach chirurgischen und pathologischen Kriterien vollständig (RO) entfernt (reseziert) wurde. Das Verfahren beinhaltet die Bestimmung und Analyse des Expressionsprofiles von 30 oder weniger Markergenen in einer Gewebeprobe des bei der Operation entfernten primären Tumors des Patienten. Mit Hilfe des Verfahrens wird für den einzelnen Patienten vorhergesagt, ob er/sie innerhalb von drei Jahren nach der Operation mit einer Progression der Krebserkrankung rechnen muss oder nicht. Als Progression der Erkrankung gilt hier die fachgerechte medizinische Feststellung (Diagnose) eines Rezidivs der Erkrankung im selben Organ, einer Tochtergeschwulst (Metastase) in anderen Organen, oder das Auftreten anderer Krebserkrankungen. Das Verfahren erlaubt, anders ausgedrückt, die Vorhersage des dreijährigen progressionsfreien Überlebens von Dickdarmkrebspatienten durch Bestimmung eines Genexpressionsprofils, das SEQ ID NO:1 umfasst. 30 Markergene sind durch ihre Sequenzen wie dargestellt in den SEQ ID NOs: 1 bis 30 definiert. Ein Aspekt der Erfindung betrifft ein spezifisches Genexpressionsprofil einer Untergruppe von sieben Genen aus den 30 Markergenen. Ein anderer Aspekt der Erfindung betrifft ein Genexpressionprofil von fünf Genen aus den 30 Markergenen. Die Genauigkeit der Vorhersage einer Progression beträgt im Fall des Expressionsprofils aus fünf Genen 82%. Ebenfalls offenbart werden Kits zur Durchführung des erfinderischen Verfahrens und diagnostische Kits. Weitere Ausführungsformen der Erfindung betreffen die die in den Ansprüchen 10-13 definierten Verwendungen.

### Hintergrund der Erfindung und Stand der Technik

Dickdarmkrebs, auch als kolorektales Karzinom bezeichnet, stellt die dritthäufigste Tumorentität in westlichen Ländern dar. In Deutschland erkranken jedes Jahr etwa 66.000 Patienten an Dickdarmkrebs. Das kolorektale Karzinom ist eine heterogene Krankheit mit komplexer Ursache. Dickdarmkrebspatienten werden aufgrund von histopathologischen Kriterien, die von der Union International Contre le Cancer (UICC) definiert wurden, in vier klinische Stadien, UICC I-IV, eingeteilt. Das TNM-Klassifikationschema der UICC wird weltweit verwendet.

Patienten mit Dickdarmkrebs im UICC Stadium I haben einen TNM-Status von T_{1/2}N₀M₀. Bei diesen Patienten sind keine regionalen Lymphknoten mit Metastasen befallen (N=O) und es sind auch keine Metastasen festgestellt und histologisch gesichert worden (M=O).

Patienten mit Dickdarmkrebs im Stadium II haben einen TNM-Status von T_{3,4}N₀M₀. Obwohl der Primärtumor deutlich größer ist als im Stadium I und auch bereits die Darmwand durchdrungen hat, sind bei diesen Patienten keine Metastasen in den regionalen Lymphknoten und keine Metastasen festgestellt worden.

Etwa die Hälfte aller neu-diagnostizierten Patienten, in Deutschland etwa 33.000 Patienten pro Jahr, haben Dickdarmkrebs im UICC Stadium I und II. Die chirurgische Komplettresektion von Tumoren in den klinischen Stadien I und II ist sehr effektiv und führt zu progressionsfreien Überlebensraten von 76% nach 5 Jahren im UICC Stadium I und 67% im UICC Stadium II. Jedoch kommt es innerhalb von 3 Jahren nach der operativen Komplettentfemung des Primärtumors bei etwa 18% der Dickdarmkrebspatienten im UICC Stadium I und bei 26% der Dickdarmkrebspatienten im UICC Stadium II zu einem Fortschreiten (Progression) der Krebserkrankung. Die Diagnose von Tochtergeschwülsten (Metastasen) des Primärtumors in Leber und/oder Lunge konstituieren die Mehrzahl der beobachteten Progressionen.

Patienten im UICC Stadium III haben einen TNM-Status T₁₋₄, N₁₋₂M₀. Für Patienten in diesem ist typisch, dass regionale Lymphknoten bereits mit Absiedelungen des Primärtumors befallen sind, jedoch noch keine Metastasen in anderen Organen festgestellt wurden. Das Vorhandensein von befallenen Lymphknoten im UICC Stadium III erhöht die Wahrscheinlichkeit der Progression der Erkrankung beträchtlich. Etwa 60% der Patienten im Stadium III müssen mit Progressionen der Erkrankung innerhalb von fünf Jahren nach der chirurgischen Entfernung des Primärtumors rechnen. Aufgrund dieser hohen Progressionsrate bekommen Patienten im UICC Stadium III nach den Richtlinien der deutschen Krebsgesellschaft eine unterstützende (adjuvante) Chemotherapie. Die adjuvante Chemotherapie verringert das Auftreten von Progressionen um etwa 10-20%, so dass in der Regel nur etwa 40-50% der Stadium III Patienten nach Operation und adjuvanter Chemotherapie innerhalb der ersten 5 Jahre eine Progression der Erkrankung erleiden.

Dickdarmkrebspatienten, bei denen bereits bei der Erstdiagnose Metastasen festgestellt und histologisch gesichert wurden, werden in das UICC Stadium IV eingeteilt. Sie haben nur eine relativ geringe 5-Jahresüberlebenswahrscheinlichkeit. In Deutschland betrifft dies etwa 20.000 Patienten. Bei diesen Patienten treten synchron oder metachron Lungen- oder Lebermetastasen auf. Bei etwa ca. 4.000 der Patienten im UICC Stadium IV sind eine Entfernung des primären Tumors und eine komplette Metastasenresektion (RO) technisch möglich, die mit einer 5-Jahres-Überlebensrate von ca. 30% einhergeht. Bei den übrigen 16.000 Patienten im UICC Stadium IV ist aus verschiedensten Gründen (multinodulär, ungünstige Lokalisation der Metastasen an Gefäßen und Gallengängen, extrahepatisch) eine Resektion nicht möglich. In diesen Fällen sind palliative Therapieoptionen indizien. Ziel der palliativen chemotherapeutischen Behandlung ist neben der Aufrechterhaltung einer guten Lebensqualität die Verlägerung der Überlebenszeit.

Es gibt eine Reihe von Problemen bei der Klassifikation und Stadiumeinteilung der Dickdarmkrebspatienten. Die Einteilung von Patienten in das Stadium I und II ist nicht genau. Etwa 10% der Stadium I Patienten und etwa 25% der Stadium II Patienten erleiden innerhalb von 5 Jahren, die Mehrzahl bereits innerhalb von zwei Jahren nach operativer Entfernung des Primärtumors eine Progression. Allein in Deutschland betrifft dies etwa 6000-8000 Patienten jährlich. Es gibt keine Möglichkeit, die Patienten mit hoher Progressionswahrscheinlichkeit aus der homogen erscheinenden Gruppe herauszufinden. Seit längerer Zeit wird in Fachkreisen darüber diskutiert, ob man Patienten im UICC Stadium II grundsätzlich eine unterstützende (adjuvante) Chemotherapie verabreicht. Wegen der relativ geringen Progressionswahrscheinlichkeit von 33% innerhalb von 5 Jahren bei Stadium II Patienten, ist der Nutzen einer solchen Therapie im Voraus nur schwer zu bestimmen und bleibt deshalb kontrovers diskutiert. Ungefähr 67% aller Patienten im Stadium II würden von einer adjuvanten Chemotherapie nicht profitieren. Die Kosten wären enorm hoch.

Basierend auf prädiktiven Markern könnte eine individuelle Therapieentscheidung gefällt werden. In diesem Zusammenhang gab es viele Versuche, neue Marker zu finden, welche Patienten mit erhöhtem Progressionsrisiko identifizieren. Hawkins et al. (2002) Gastroenterology 122:1376-1387 untersuchten in diesem Zusammenhang Mikrosatelliteninstabilität und Promotermethylierung. Noura et al. (2002) J Clin Oncol 20:4232-benutzten einen RT-PCR basierten Nachweis von Lymphknotenmikrometastasen. Zhou et al. (2002) Lancet 359:219-225 untersuchten Allelungleichgewichte, um Rezidive in kolorektalen Karzinomen vorher sagen zu können. Eschrich et al. (2005) J Clin Oncol. 2005 May 20;23(15):3526-35 benutzten cDNA Microarrays, um die Überlebenswahrscheinlichkeit von Patienten mit kolorektalern Krebs vorherzusagen.

CRONER ROLAND S ET AL: "Microarray versus conventional prediction of lymph node metastasis in colorectal carcinoma" CANCER, AMERICAN CANCER SOCIETY, PHILADELPHIA, PA, US, Bd. 104, Nr. 2, 10. Juni 2005 (2005-06-10), Seiten 395-404, ISSN: 0008-543X beschreibt ein auf der Anwendung eines Mikroarrays basiertes Verfahren zur Bestimmung der Genexpression in primärem Tumormaterial von einem aus Patienten der Klasse UICC I und II entfernten Karzinom und die Anwendung der Genexpressionsdaten zur Vorhersage von Metastasen in den Patienten. Obwohl das Dokument keine spezifischen Gene erwähnt, ist es klar, dass eine Verbesserung der Vorhersage durch die Bestimmung der Genexpressionsprofile erzielt wird (siehe Seite 400, rechte Spalte).

WANG Y ET AL: "Gene expression profiles and molecular markers to predict recurrence of Dukes' B colon cancer" JOURNAL OF CLINICAL ONCOLOGY, GRUNE AND STRATTON, NEW YORK, NY, US, Bd. 22, Nr. 9, 1. Mai 2004 (2004-05-01), Seiten 1564-1571, XP003014769 ISSN: 0732-183X beschreibt ein auf der Verwendung eines Genechips basiertes Verfahren zur Bestimmung der Genexpression in primäres Tumormaterial von aus 74 Patienten der Klasse Dukes Typ B entfernten Karzinomen. Mit den Daten werden Gene identifiziert die als Signatur für die Prognose der Patienten eingesetzt werden können. Die Patienten werden regelmässig nach Metastasen untersucht. Es wird gezeigt dass die Gensignatur mit einer Sensitivität von 72% und einer Spezifizität von 83% die Rezidiven und Metastasen vorhersagen können.

WO 20041031775 A (ONCOTHERAPY SCIENCE INC [JP]; JAPAN AS REPRESENTED BY THE PR [JP]; NAK) 15. April 2004 (2004-04-15) (siehe Anspruch 1) beschreibt ein auf Bestimmung der Genexpression basierendes Verfahren zur Vorhersage der Wahrscheinlichkeit, dass Metastasen sich in einem Patienten mit einem kolorektalen Karzinom entwickeln.

Allen in der Literatur untersuchten Markern ist gemeinsam, dass sie bisher nicht als Grundlage prognostischer Assays in der klinischen Praxis angewendet werden, da sie nicht unabhängig validiert sind. Eine mögliche Ursache dafür könnte darin liegen, dass die Progression des kolorektalen Karzinoms eine Folge verschiedenster genetischer Ereignisse ist, welche innerhalb des malignen Epithels stattfinden, oder durch modifizierende Ereignisse des umgebenden stromalen Gewebes induziert werden. Um die potentielle Komplexität des Fortschreitens der Erkrankung verstehen zu können, bedarf es einer umfassenderen Analyse der zu Grunde liegenden molekularen Ereignisse.

### Technisches Problem der Erfindung

Das der Erfindung zugrunde liegende technische Problem besteht in der Bereitstellung einer verlässlichen Diagnostik, die zu verbesserten individuellen Therapien führen kann.

Das technische Problem ist durch die Bereitstellung der hierin offenbarten Ausführungsformen und im Besonderen durch die die Erfindung charakterisierenden Ansprüche gelöst. Die Erfindung umfasst daher ein Verfahren zur Vorhersage der Wahrscheinlichkeit einer Progression (Lokalrezidiv, Metastasen, Zweitmalignomen) innerhalb der ersten drei Jahre nach der chirurgischen Entfernung der primären Tumoren von Darmkrebspatienten im UICC Stadium I und UICC Stadium II.

Die Erfindung bezieht sich auf die Bestimmumg von Expressionsprofilen bestimmter Gene, die bei Karzinomen insbesondere gastro-intestinalen und besonders bevorzugt bei primären kolorektalen Karzinomen von Bedeutung sind. In diesem Zusammenhang lehrt die Erfindung ein Testsystem zur (*in vitro*) Detektion des Progressionsrisikos eines vorstehend genannten Karzinoms, enthaltend eine Methode zur quantitativen Messung des Expressionsprofils bestimmter Markergene in bestimmten Tumorgewebeproben sowie bioinformatische Auswertemethoden, um daraus die Wahrscheinlichkeit des Auftretens einer Progression (Lokalrezidiv, Metastasen, Zweitmalignomen) für einen Patienten zu berechnen, bei welchem ein Kolorektales Karzinom im UICC Stadium I oder UICC Stadium II diagnostiziert und behandelt wird. Die 30 Markergene sind insbesondere in der Tabelle 1 definiert und werden durch ihre entsprechenden Sequenzen oder weitere synonyme Identifikatoren in der Tabelle charakterisiert. Dabei handelt es sich um: RAB22A (RAB22A, member RAS oncogene family) [Affymetrix Nummer 218360_at] SEQ_ID_1, NDRG3 (NDRG family member 3) [Affymetrix Nummer 217286_s_at] SEQ_ID_2, UBL5 (ubiquitin-like 5) [Affymetrix Nummer 218011_at] SEQ_ID_3, MRPL22 (mitochondrial ribosomal protein L22) [Affymetrix Nummer 218339-ast] SEQ_ID_4, HLA-DQA1 (major histocompatibility complex, class II, DQ alpha 1) [Affymetrix Nummer 212671_s_at] SEQ_ID_5, C10orf116 (chromosome 10 open reading frame 116) [Affymetrix Nummer 20357 1_s_at] SEQ_ID_6, PAM (peptidylglycine alpha-amidating monooxygenase) [Affymetrix Nummer 212958_x_at] SEQ_ID_7, TM4SF20 (transmembrane 4 L six family member 20) [Affymetrix Nummer 220639_at] SEQ_ID_8, HLA-DPA1 (major histocompatibility complex, class II, DP alpha 1) [Affymetrix Nummer 211991_s_at] SEQ_ID_9, PAM (peptidylglycine alpha-amidating monooxygenase) [Affymetrix Nummer 202336_s_at] SEQ_ID_10, ADAM9 (ADAM metallopeptidase domain 9 (meltrin gamma)) [Affymetrix_Nummer 202381_at] SEQ_ID_11, RAB22A (RAB22A, member RAS oncogene family) [Affymetrix Nummer 213405_at] SEQ_ID_12, CH25H (cholesterol 25-hydroxylase) [Affymetiix Nummer 206932_at] SEQ_ID_13, NDUFB11 (NADH dehydrogenase (ubiquinone) 1 beta subcomplex, 11, 17.3kDa) [Affymetrix Nummer 218320_s_at] SEQ_ID_14, LSM7 (LSM7 homolog, U6 small nuclear RNA associated (S. cerevisiae)) [Affymetrix Nummer 204559_s_at] SEQ_ID_15, ITGB3BP (integrin beta 3 binding protein (beta3-endonexin)) [Affymetrix Nummer 205176_s_at] SEQ_ID_16, RPL36A (ribosomal protein L36a) [Affymetrix Nummer 201406_at] SEQ_ID_17, SNRPE (small nuclear ribonucleoprotein polypeptide E) [Affymetrix Nummer 203316_s_at] SEQ_ID_18, HLA-DRB1 (major histocompatibility complex, class II, DR beta 1) [Affymetrix Nummer 209312_x_at] SEQ_ID_19, HLA-DQB1 (major histocompatibility complex, class II, DQ beta 1) [Affymetiix Nummer 209823_x_at] SEQ_ID_20, HEATR1 (HEAT repeat containing 1) [Affymetrix Nummer 218594_at] SEQ_ID_21, IFI44L (interferon-induced protein 44-like) [Affymetrix Nummer 204439_at] SEQ_ID_22, FLJ10986 (NA) [Affymetrix Nummer 219718_at] SEQ_ID_23, HLA-DRB5 (major histocompatibility complex, class II, DR beta 5) [Affymetrix Nummer 204670_x_at] SEQ_ID_24, ADRM1 (adhesion regulating molecule 1) [Affymetrix Nummer 201281_at] SEQ_ID_25, IFT20 (intraflagellar transport 20 homolog (Chlamydomonas)) [Affymetrix Nummer 210312_s_at] SEQ_ID_26, CES1 (carboxylesterase 1 (monocyte/macrophage serine esterase 1)) [Affymetrix Nummer 209616_s_at] SEQ_ID_27, CLDN15 (claudin 15) [Affymetrix Nummer 219640_at] SEQ_ID_28, BLCAP (bladder cancer associated protein) [Affymetrix Nummer 201032_at] SEQ_ID_29, ROBO1 (roundabout, axon guidance receptor, homolog 1 (Drosophila)) [Affymetrix Nummer 213194_at] SEQ_ID_30.

Die Vorhersage (Prädiktion) von Progressionen eines primären kolorektalen Karzinoms ist für den Kliniker von besonderer Relevanz, da es die weitere Behandlung des Patienten entscheidend beeinflussen kann. Finden sich keine Tumorabsiedelungen, weder in regionäre Lymphknoten noch Metastasen, handelt es sich um das UICC Stadium I oder II. Diese Tumore werden, wenn es kolorektale Karzinome sind, ausschließlich mittels Operation behandelt. Eine adjuvante Chemotherapie ist (außerhalb klinischer Studien) nicht vorgesehen. Finden sich dagegen Tumorzellen in regionären Lymphknoten (UICC Stadium III), so wird gemäß den Leitlinien der Deutschen Krebsgesellschaft und anderer internationaler Gesellschaften eine postoperative adjuvante Chemotherapie empfohlen. Durch die ajduvante Chemotherapie ergibt sich ein progressionsfreies 3 Jahres Überleben der Patienten im UICC Stadium III von etwa 69 %, ohne anschließende Chemotherapie liegt das 3 Jahres progressionsfreie Überleben nur bei etwa 49 %. Auch das Gesamtüberleben wird durch die adjuvanten Chemotherapie signifikant beeinflusst. Beim Rektumkarzinom ist es ebenfalls von entscheidender Bedeutung, ob bereits Tumorzellen in regionären Lymphknoten vorliegen. In diesen Fällen wird die präoperative Radiochemotherapie empfohlen, da sie das Auftreten von Lokalrezidiven im Rektum signifikant vermindert. Zudem können durch eine präoperative Radiochemotherapie signifikant mehr Patienten kontinenzerhaltend operiert werden, was bei diesen Patienten zu einer wesentlichen Verbesserung der postoperativen Lebensqualität beiträgt.

Im Zusammenhang mit dieser Erfindung bezeichnet der Begriff "kolorektales Karzinom" insbesondere polypoide, plateauförmige, ulzeröse und flächenhafte (szirrhöse) Formen, die histologisch in solide, schleimbildende oder drüsige Adenokarzinome, Siegelringzellenkarzinome, squamöse, adenosquamöse, kribriforme, plattenepithelartige oder undifferenzierte Karzinome entsprechend der WHO- Klassifikation typisiert werden. (Becker, Hohenberger, Junginger, Schlag. Chirurgische Onkologie. Thieme, Stuttgart 2002).

Im Zusammenhang mit der Erfindung umfasst der Begriff "Genexpressionsprofil" sowohl die Bestimmung von "Expressionsprofilen", als auch der einzelnen "Expressionsniveaus" der entsprechenden Gene. Der Begriff "Expressionsniveau", als auch der Begriff "Expressionsprofil" umfasst erfindungsgemäß sowohl die Quantität des Genprodukts, als auch seine qualitative Veränderungen wie z. B. Methylierungen, Glykosylierungen, Phosphorylierungen u.s.w. Somit wird bei der Bestimmung des "Expressionsprofils" im Zusammenhang mit der Erfindung im Wesentlichen auf die Quantität der entsprechenden Genprodukte (RNA/ Protein) geachtet. Das Expressionsniveau wird gegebenenfalls mit dem anderer Individuen verglichen. Entsprechende Ausführungsbeispiele sind im experimentellen Teil belegt und auch insbesondere in den Tabellen dargestellt.

Die Bestimmung der Expressionsprofile der hierin dargestellten Gene (Genabschnitte) wird insbesondere in Geweben und/oder einzelnen Zellen des Gewebes durchgeführt. Verfahren zur Bestimmung des Expressionsprofils umfassen daher (im Sinne dieser Erfindung) z. B. *in situ*-Hybridisierungen, PCR basierte Methoden (z. B. Taqman) oder Mikroarray basierte Verfahren (siehe auch experimenteller Teil der Erfindung).

In einer besonderen Ausführungsform umfasst die Erfindung das oben genannte Verfahren, wobei das Expressionsprofil von mindestens dem Markergen gemäß SEQ ID NO 1 oder einem Markergen, das mindestens 90 % Identität zu dem Markgergen gemäß SEQ ID NO 1 aufweist, bestimmt wird.

In einer weiteren bevorzugten Ausführungsform umfasst die Erfindung das oben genannte Verfahren, wobei das Expressionsprofil von sieben Markergenen, wie in SEQ ID NO 1 bis SEQ ID NO 7 dargestellt, bestimmt wird.

In einer weiteren besonders bevorzugten Ausführungsform umfasst die Erfindung das oben genannte Verfahren, wobei das Expressionsprofil von einer beliebigen Kombinationen aus dem Subset der fünf Markergene, wie in SEQ ID NO 1 bis SEQ ID NO 5 dargestellt, bestimmt wird, wobei das Markergen mit einer Sequenz gemäß SEQ ID NO 1 oder das Markgergen, das mindestens 90 % Identität zu dem Markergen mit einer Sequenz gemäß SEQ ID NO 1 in dem Expressionsprofil umfasst ist.

Wie im Weiteren definiert wird, umfasst der Begriff Markergene im Sinne dieser Erfindung nicht nur die spezifischen Gensequenzen (oder die entsprechenden Genprodukte) wie in den spezifischen Nucleotidsequenzen dargestellt, sondern auch Gensequenzen, die eine hohe Homologie zu diesen Sequenzen aufweisen. Weiterhin sind die revers-komplementären Sequenzen der definierten Markergene eingeschlossen. Hochhomologe Sequenzen umfassen Sequenzen, die mindestens 90%, am meisten bevorzugt mindestens 95% homolog zu den in den SEQ ID NOS: 1 bis 30 dargestellten Sequenzen sind.

Der Begriff Markergen umfasst ein Gen oder einen Genabschnitt, der mindestens 90 % homolog, mehr bevorzugt mindestens 95 % homolog, mehr bevorzugt mindestens 98 % homolog, am meisten bevorzugt mindestens 100 % homolog zu den in den SEQ ID NO 1 bis SEQ ID NO 30 dargestellten Sequenzen in Form von Desoxyribonnukleotiden oder entsprechenden Ribonunkleotiden bzw. den daraus abgeleiteten Proteinen ist.

Die Sequenzidentität kann konventionell durch Verwendung von Computerprogrammen wie z. B. dem FASTA-Programm (W. R. Pearson (1990) Rapid and Sensitive Sequence Comparison with FASTP and FASTA Methods in Enzymology 183:63 - 98.), download z. B. als Service des EBI in Hinxton, bestimmt werden. Bei der Anwendung von FASTA oder einem anderen Sequenz- Alignment-Programm zur Bestimmung, ob eine bestimmte Sequenz beispielsweise zu 95 % identisch ist mit einer Referenzsequenz der vorliegenden Erfindung, werden die Parameter vorzugsweise so eingestellt, dass der Prozentanteil der Identität über die gesamte Länge der Referenzsequenz berechnet wird und dass Homologielücken (so genannte gaps) von bis zu 5 % der Gesamtzahl der Nukleotide in der Referenzsequenz erlaubt sind. Wichtige Programmparameter wie z. B. GAP PENALTIES und KTUP werden auf den Standardwerten belassen.

In einer besonderen Ausführungsform können die entsprechenden Markergene nicht nur in Tumor- sondern auch in anderen biologischen Proben, wie z.B. im Blut, Blutserum, Blutplasma, Stuhl oder anderen Körperflüssigkeiten (Bauchhöhlen-Ascites, Lymphflüssigkeit) bestimmt werden. Dementsprechend ist die vorliegende Erfindung nicht limitiert auf die Untersuchung von gefrorenem oder frischem Tumorgewebe. Die erfindungsgemäßen Ergebnisse können ebenfalls durch die Untersuchung von fixiertem Tumorgewebe zum Beispiel Paraffinmaterial, erhalten werden. In fixiertem Material werden insbesondere auch vorzugsweise andere Detektionsverfahren zum Nachweis der Gene/Genexpressionsprodukte genutzt, z.B. RNA spezifische Primer im Rahmen einer real-time PCR.

Wie auch in den Ausführungsbeispielen dargestellt, wird das Expressionsprofil der hier offenbarten 30 Markergene (oder einer Auswahl daraus), vorzugsweise, durch die Messung der Quantität der mRNA der Markergene bestimmt. Diese Quantität der mRNA der Markergene kann z.B. mittels Genchiptechnologie, (RT-) PCR (zum Beispiel auch an fixiertem Material), Northern Hybridisierung, Dot-Blotting oder in situ Hybridisierung bestimmt werden. Weiterhin kann das erfinderische Verfahren auch durchgeführt werden, indem der Fachmann die Genprodukte auf Protein- oder Peptidebene misst. Somit umfasst die Erfindung auch die hier beschriebenen Verfahren, bei denen die Genexpressionsprodukte in Form ihrer synthetisierten Proteine (oder Peptide) bestimmt werden. Hierbei kann sowohl die Quantität, als auch die Qualität (z.B. Modifikationen, wie Phosphorylierungen oder Glycosylierungen) bestimmt werden. Vorzugsweise wird in diesem Zusammenhang jedoch das Expressionsprofil der Markergene durch die Messung der Polypeptidquantität der Markergene bestimmt und, falls gewünscht, mit einem. Referenzwert der jeweiligen Vergleichsprobe(n) verglichen. Die Polypeptidquantität der Markergene kann durch ELISA, RIA, (Immuno-) Blotting, FACS oder immunhistochemische Methoden bestimmt werden.

Die in der vorliegenden Erfindung besonders vorzugsweise verwendete Mikroarray Technologie erlaubt die gleichzeitige Messung des mRNA Expressionsniveaus von vielen Tausend Genen und stellt somit ein wichtiges Hilfsmittel dar, um differentielle Expression zwischen zwei biologischen Proben oder Gruppen von biologischen Proben zu bestimmen. Analysen können aber auch, wie dem Fachmann bekannt, durch einzelne reverse Transkriptase-PCR, kompetetive PCR, "real time PCR", "differential display RT-PCR", Northern Blot- Analyse und andere verwandte Verfahren durchgeführt werden.

Am Besten ist es jedoch, die komplementäre DNA (cDNA) bzw. komplementäre RNA (cRNA), welche ausgehend von der zu untersuchenden RNA produziert wird, mit Hilfe von Mikroarrays zu untersuchen. Eine große Zahl von verschiedenen Arrays sowie deren Herstellung sind dem Fachmann bekannt und beispielsweise in den US Patenten 5,445,934; 5,532,128; 5,556,752; 5,242,974; 5,384,261; 5,405,783; 5,412,087; 5,424,186;5,429,807; 5,436,327; 5,472,672; 5,527,681; 5,529,756; 5,54533 1; 5,554,501; 5,561,071;5,571,639; 5,593,839; 5,599,695; 5,624,711; 5,658,734; und 5,700,637 dargestellt.

Die wohl definierte Abfolge von Analysenschritten führt letztendlich zur Entdeckung der Markergensignaturen, mit denen die Test- von der Kontrollgruppe unterscheidenden werden kann. Diese bisher in dieser Form noch nicht beschriebene Methode setzt sich wie in den

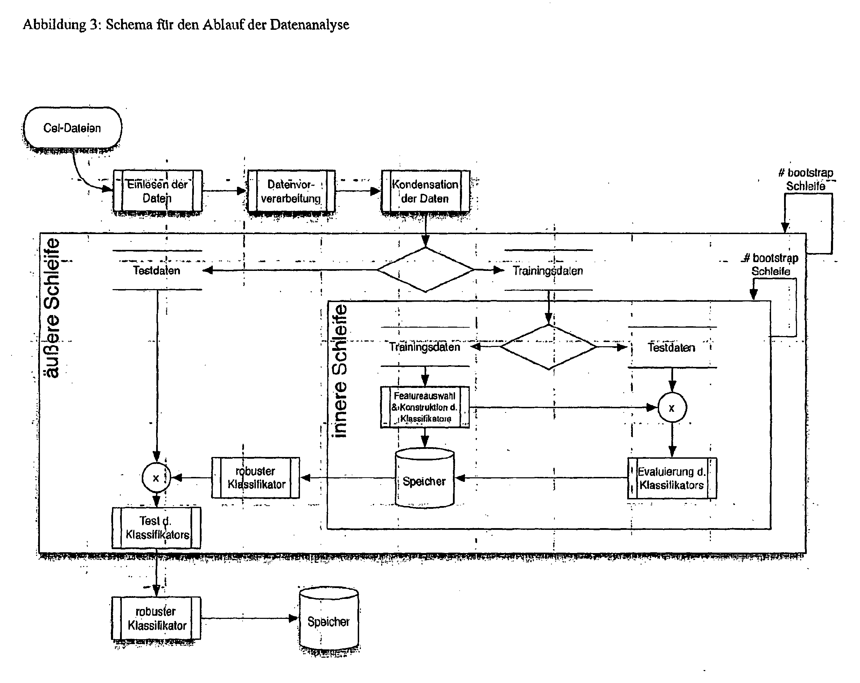

Ausführungsbeispielen detaillierter beschrieben und in Abbildung 3 skizziert wie folgt zusammen:

Die Rohdaten der Biochips werden zunächst mit FARMS wie von von Hochreiter (2006), Bioinformatics 22(8):943-9, gezeigt kondensiert und anschließend in einem zweifach geschachtelten Bootstrap-Ansatz, [Efron (1979) Bootstrap Methods - Another Look at the Jackknifing, Ann. Statist. 7, 1-6, in der äußeren Schleife in Test- und Trainigsdaten aufgeteilt. In der inneren Bootstrap-Schleife wird für die Trainingsdaten durch eine Decision-Tree-Analyse die Featurerelevanz aus den Daten extrahiert. Dazu werden in mehreren Bootstrap-Iterationen jeweils eine bestimmte Zahl von zu klassifizierenden Proben zufällig gezogen und der Einfluss eines Features aus dessen Beitrag zum Klassifikationsfehler ermittelt: Steigt der Fehler durch Permutieren der Werte eines Features, während die Werte aller anderen Features unverändert blieben, so wird dieses stärker gewichtet. Anhand einer Häufigkeitstabelle werden die am häufigsten gewählten Features ermittelt und für die Klassifikation der Testdaten durch eine Support-Vector-Machine oder anderweitige dem Fachmann bekannte Klassifikationsalgorithmen wie z.B. Kassifikations- und Regressionsbäume, Penalized Logistic Regression, Sparse Linear Discriminant Analysis, Fisher Linear Discriminant Analysis, K-Nearest Neighbors, Shrunken Centroids und Artificial Neural Networks in der äußeren Bootstrap-Schleife verwendet.

Ein Feature stellt in diesem Zusammenhang einen bestimmten Messpunkt für ein zu untersuchendes Gen dar, welches sich auf der Biochipoberfläche befindet und mit der zu untersuchenden markierten Probe hybridisiert und somit ein Intenstätssignal liefert. Die vorliegende Erfindung betrifft ebenfalls die Verwendung eines Kits zur Durchführung der hier beschrieben Verfahren, wobei das Kit spezifische DNA- oder RNA-Sonden, Primer (auch Primerpaare) zur Bestimmung von mindestens SEQ ID NO: 1 umfasst. Das Kit ist vorzugsweise ein diagnostisches Kit. Als Kit im Sinne der Erfindung wird auch ein beliebiges Mikroarray oder im Speziellen ein "Affymetrix-Genchip" bezeichnet. Das Kit kann alle oder einige der für die Durchführung des Assays benötigten Materialien sowie die erforderlichen Instruktionen enthalten.

Offenbart sind auch Darstellungen der Markergensignaturen, welche für die Behandlung, die Diagnose und die Prognose der oben genannten Erkrankungen nützlich sind. Diese Genprofildarstellungen sind auf Medien reduziert, welche maschinenlesbar sind wie z.B. computerlesbare Medien (magnetische Medien, optische Medien u.s.w.). Sie können CD ROMs sein, welche Computerprogramme für den Vergleich mit dem gespeicherten 30-Gen-Expressionsprofil, welches oben beschrieben wurde, enthalten. Ebenso können die Gegenstände digital gespeicherte Expressionsprofile enthalten, sodass diese mit Expressionsdaten von Patienten verglichen werden können. Alternativ können solche Profile in einem anderen gegenständlichem Format gespeichert werden. Eine grafische Darstellung ist beispielsweise ein solches Format.

Im Folgenden soll die Erfindung genauer auf der Basis der Sequenzen, Tabellen und Beispiele beschrieben werden, ohne darauf begrenzt zu werden.

Die Tabellen zeigen:
Tabelle 1 enthält die 30 Markergene, die in der vorliegenden Erfindung differentiell zwischen Patienten mit und ohne Progression innerhalb von drei Jahren nach der Operation des primär aufgetretenen kolorektalen Karzinoms exprimiert sind.
Tabelle 2 zeigt die Maßzahlen der Klassifikation des dreijährigen progressionsfreien Überlebens für das ausgewählte Patientenkollektiv (insgesamt 55 davon 18 mit Progression) in Abhängigkeit von der verwendeten Markergenanzahl.

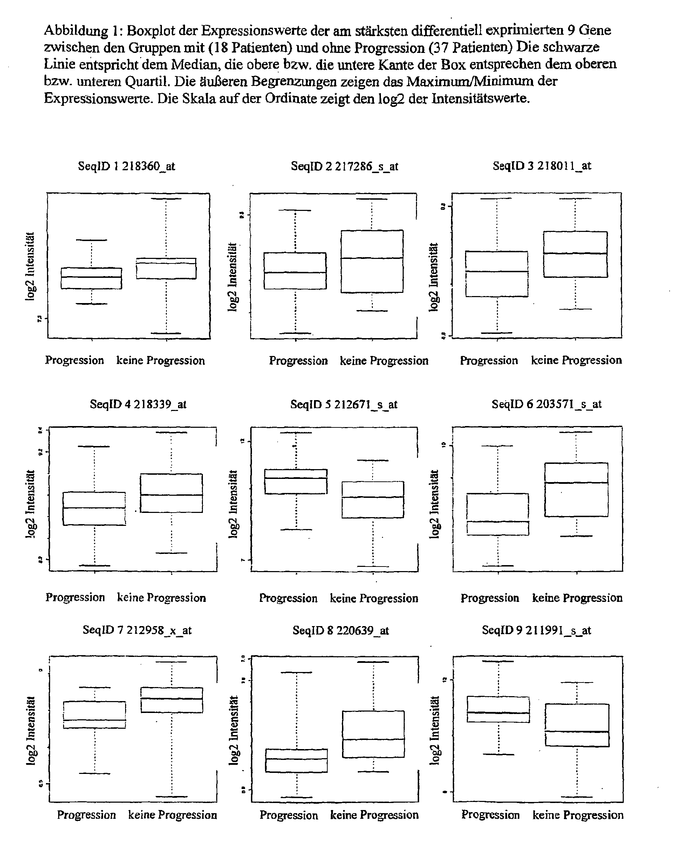

Abbildung 1 zeigt den Boxplot der Expressionwerte der besten 9 Gene aus der Markergenliste für die Patientenaruppen mit oder ohne Progression innerhalb der ersten drei Jahre nach der Operation.
Abbildung 2 zeigt die Vorhersagewahrscheinlichkeit für das Auftreten einer Progression innerhalb von drei Jahren nach einer primären Diagnose eines kolorektalen Kazinoms in Abhängigkeit von der verwendeten Markergenanzahl.
Abbildung 3 zeigt schematisch das methodischeVorgehen, welches zur Erstellung des Markergenprofils führt.

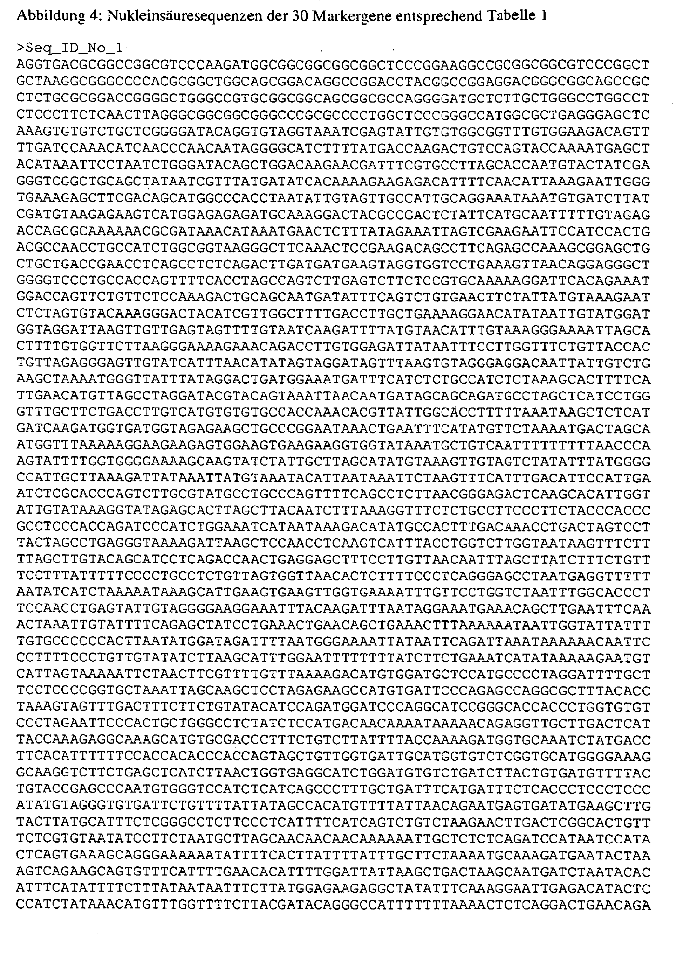

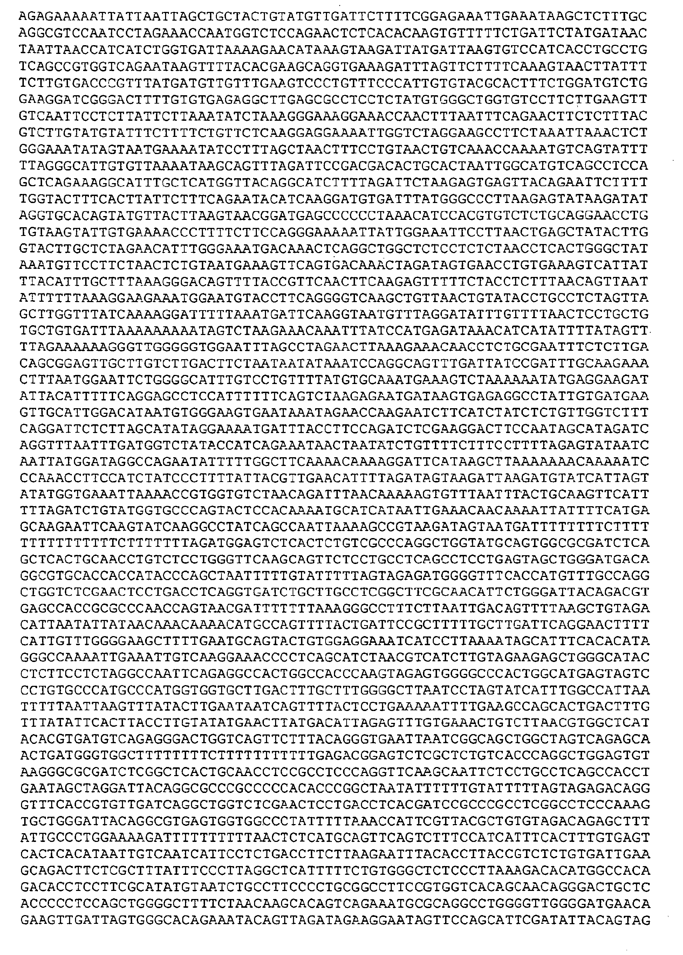

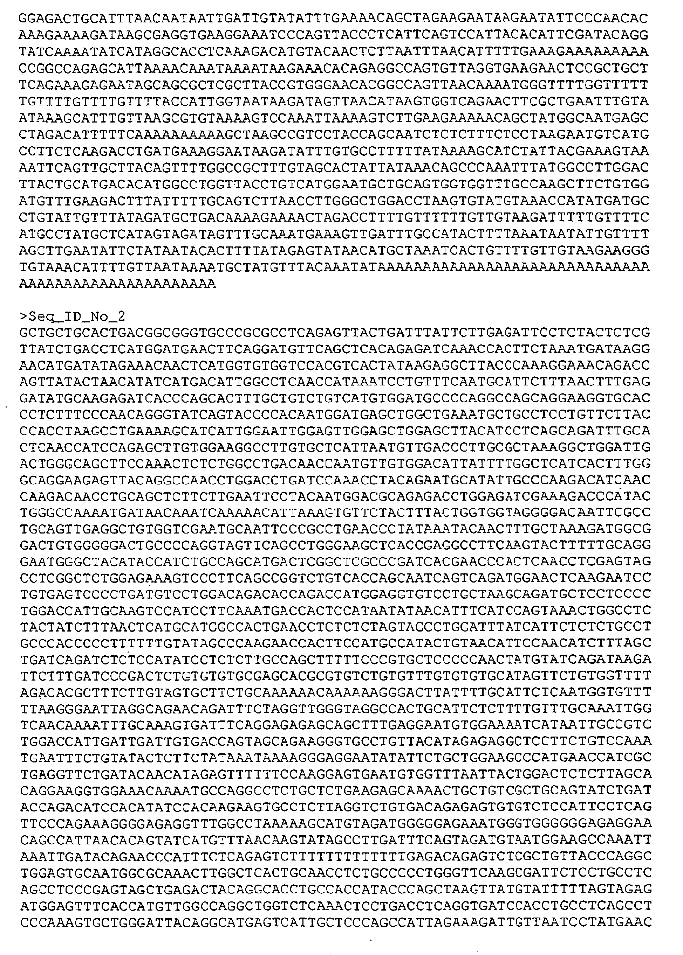

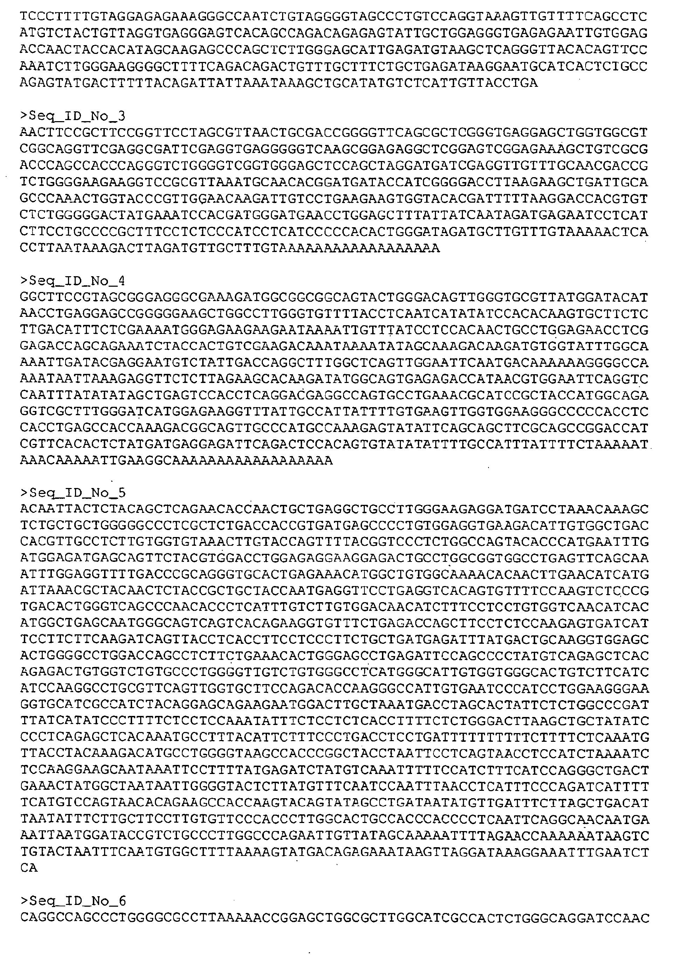

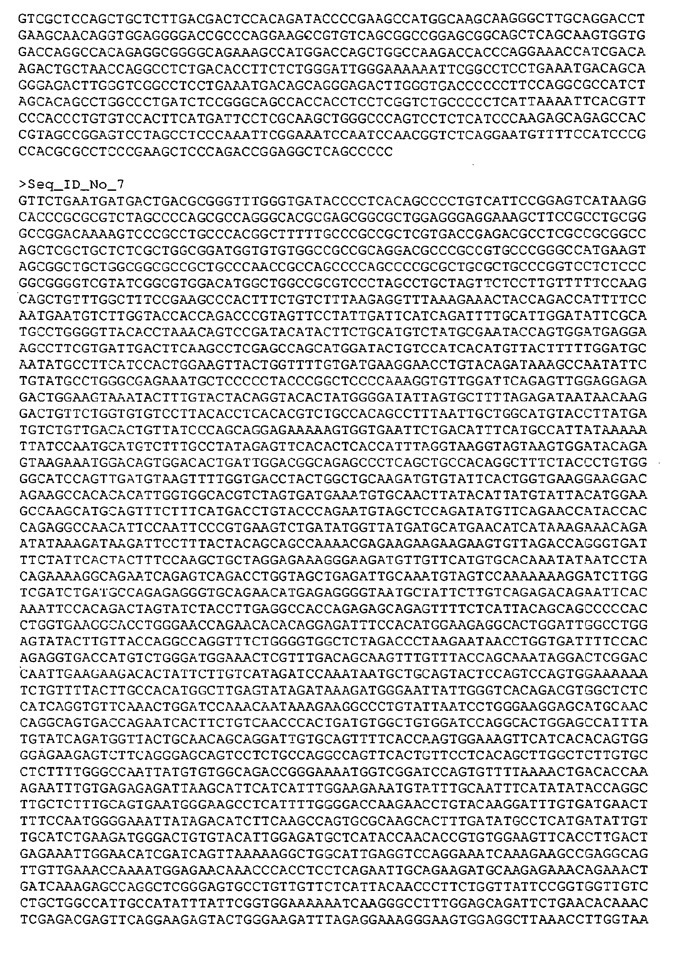

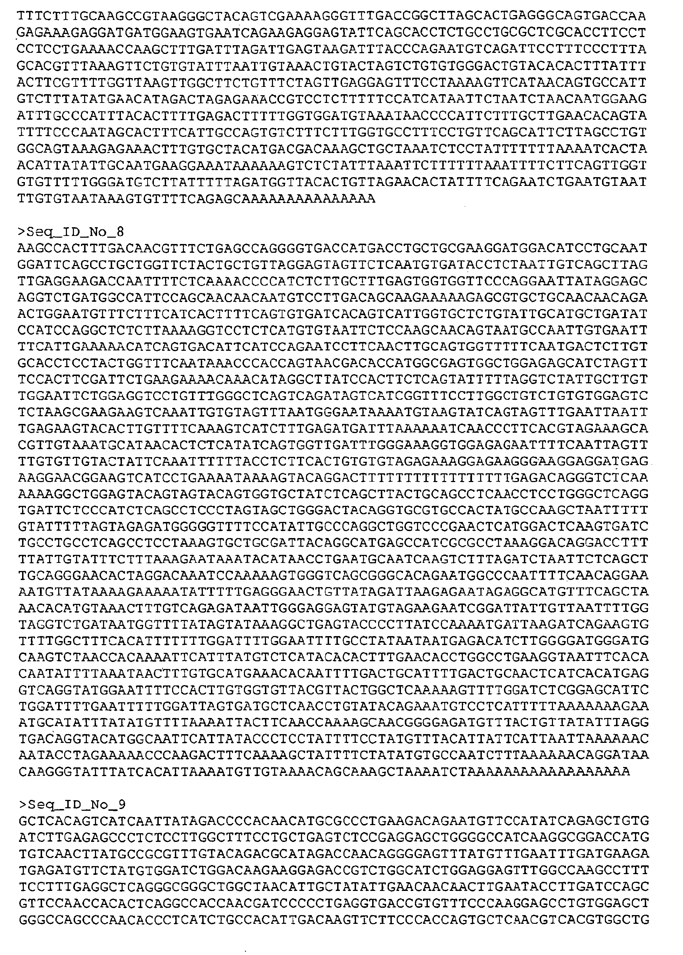

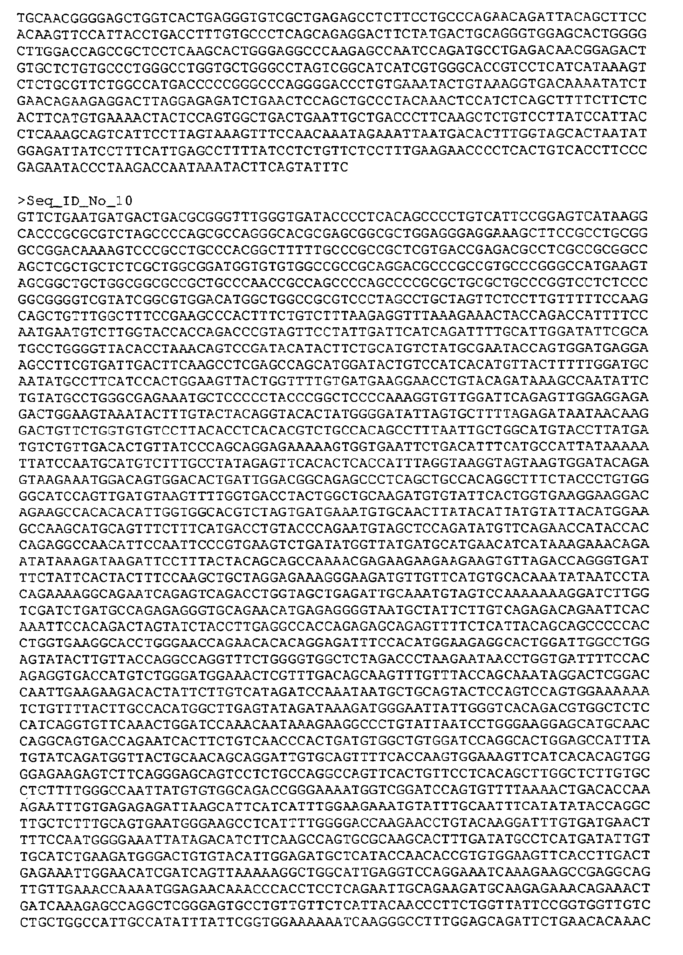

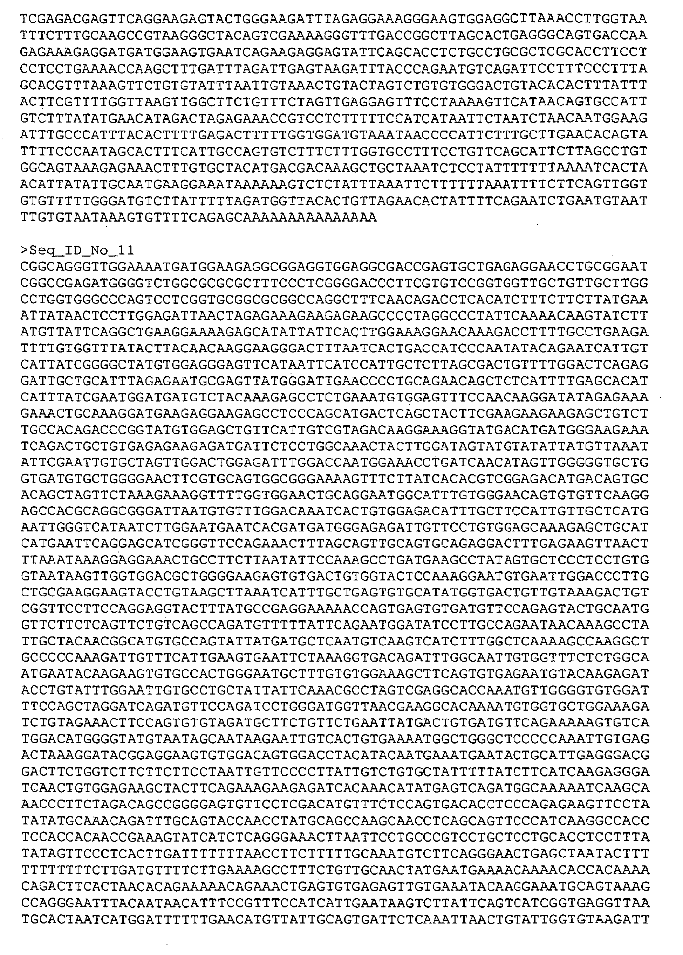

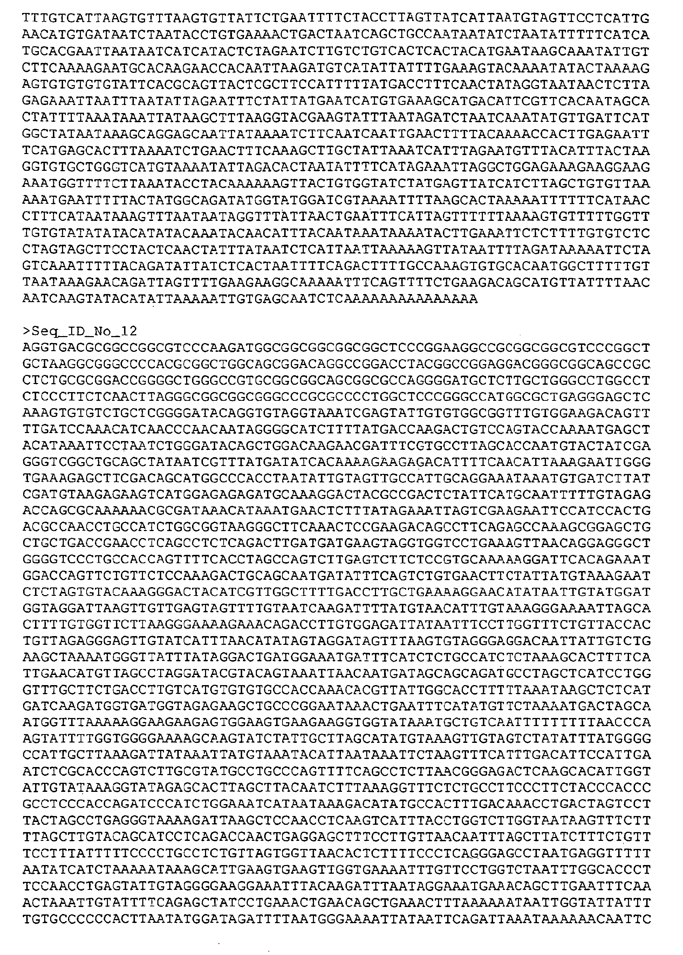

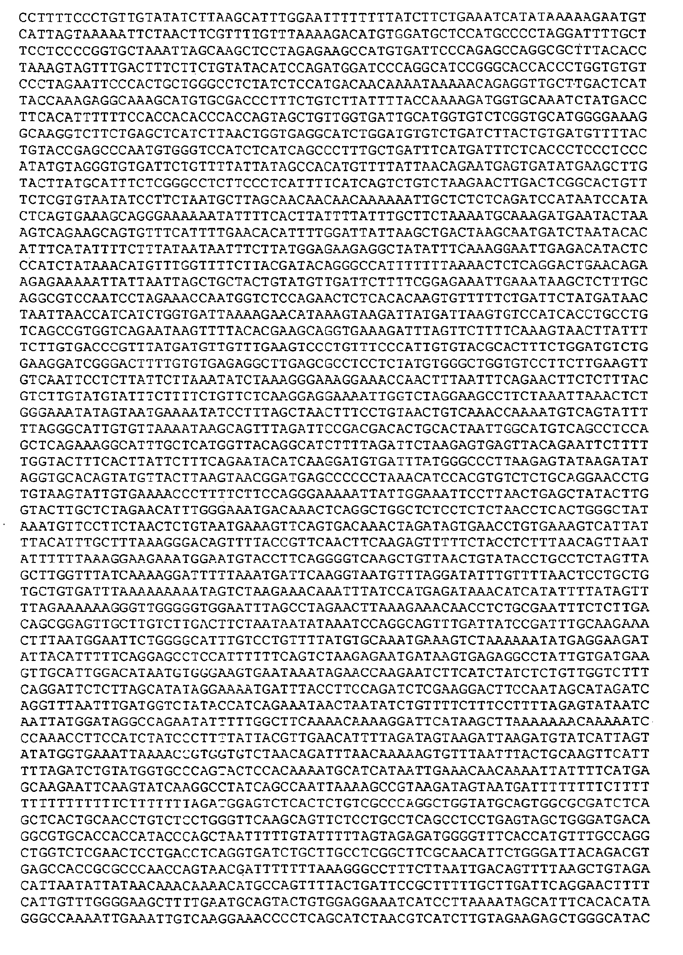

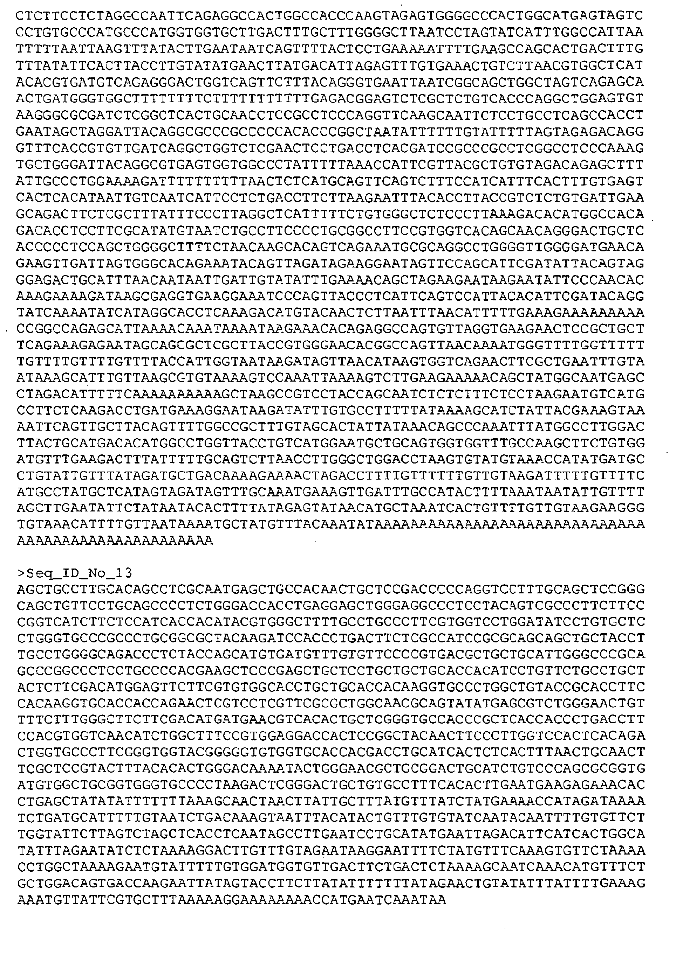

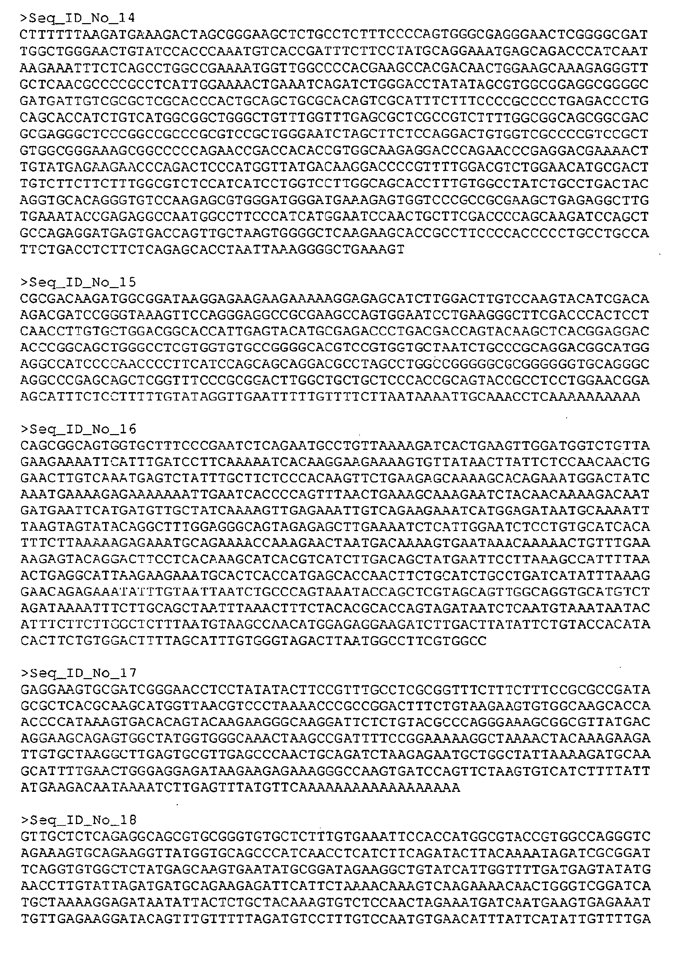

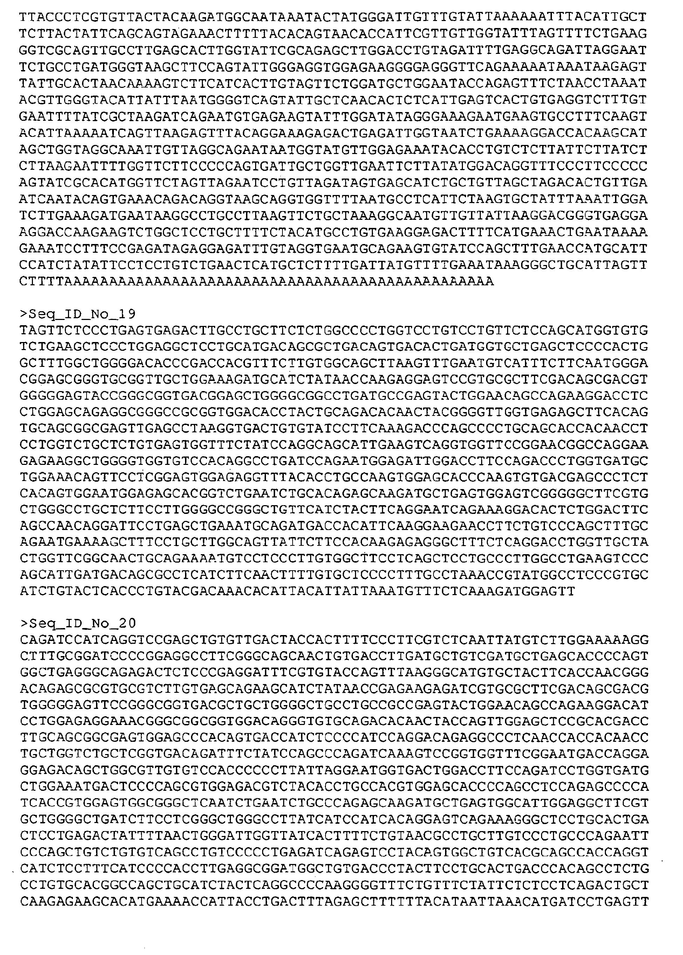

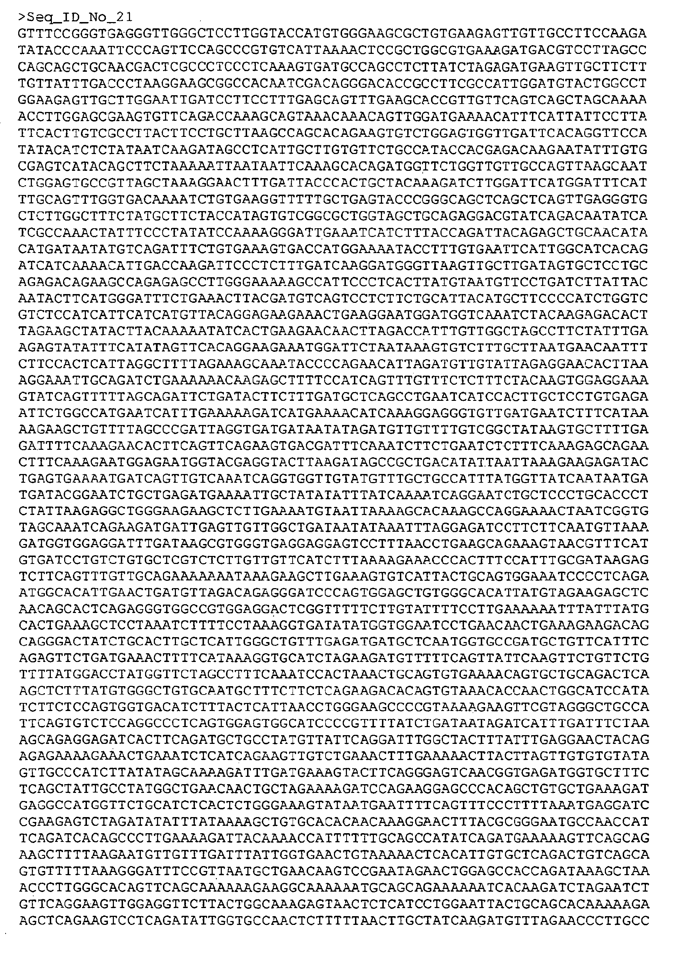

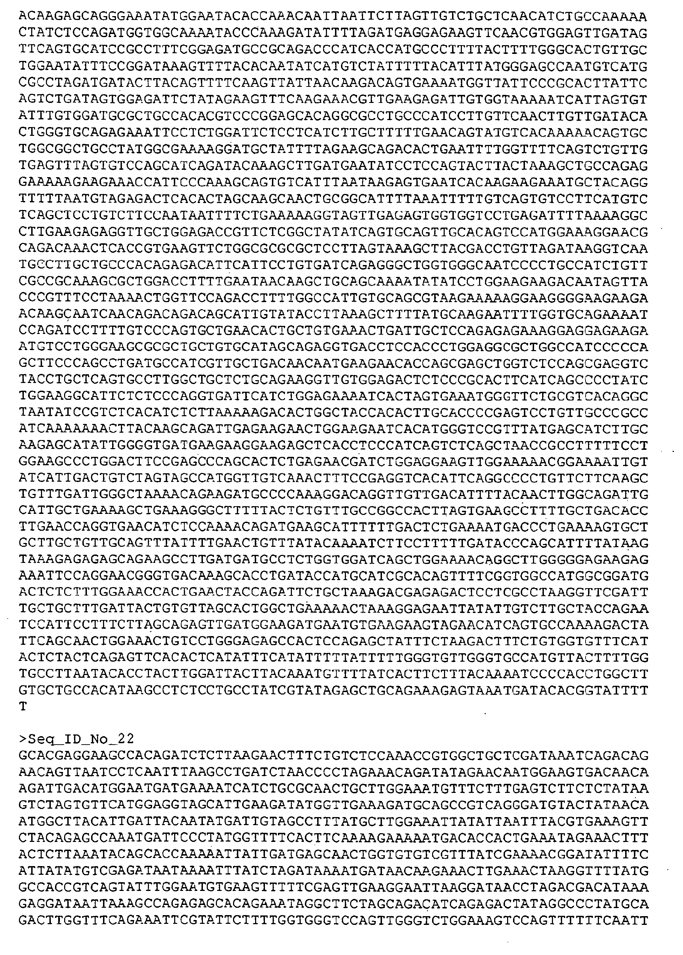

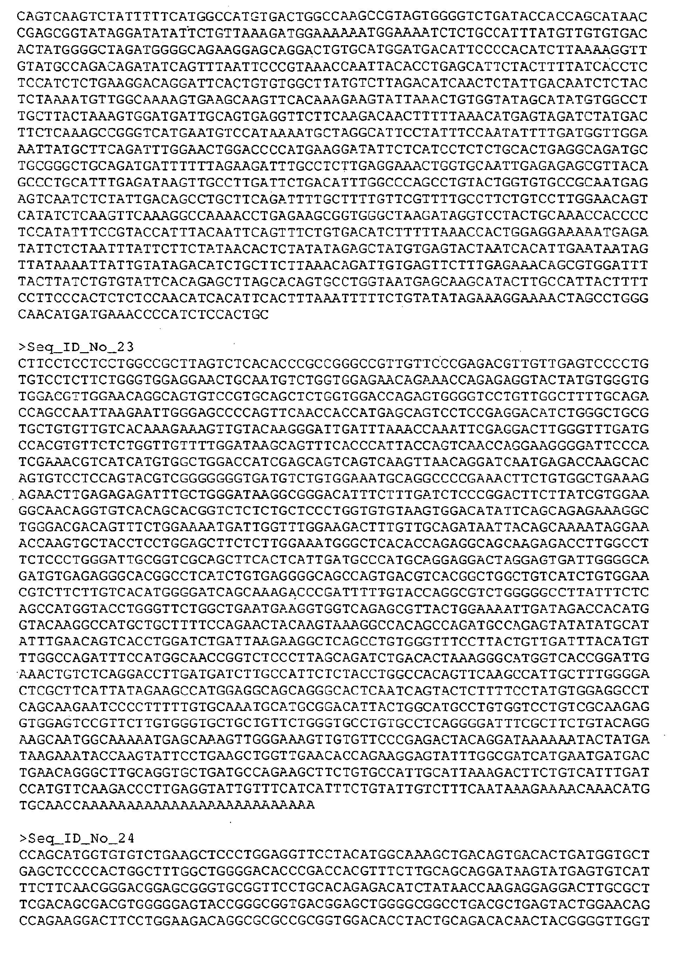

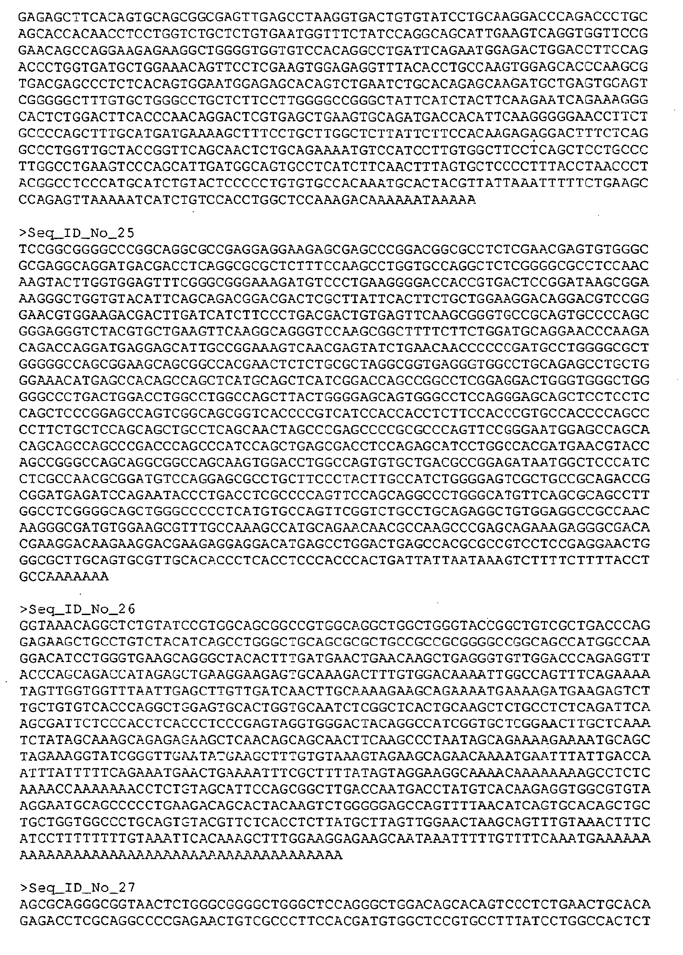

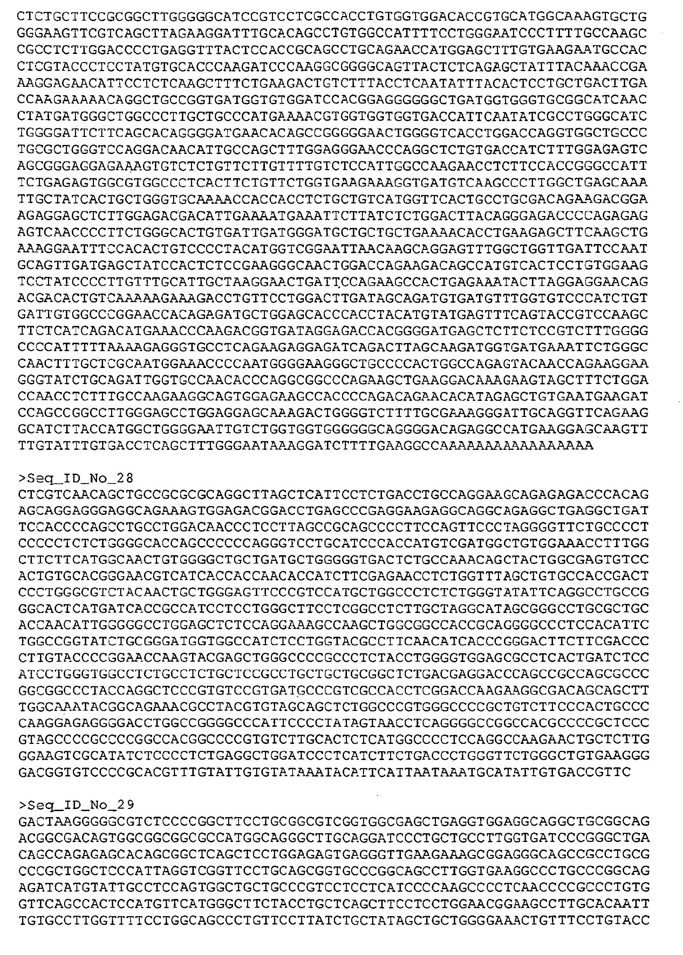

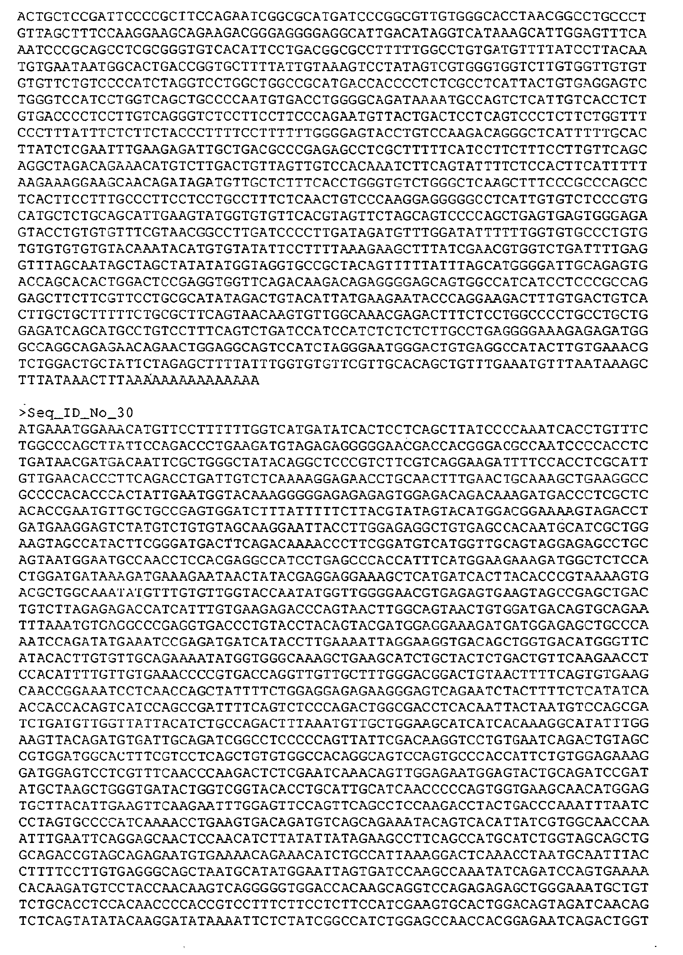

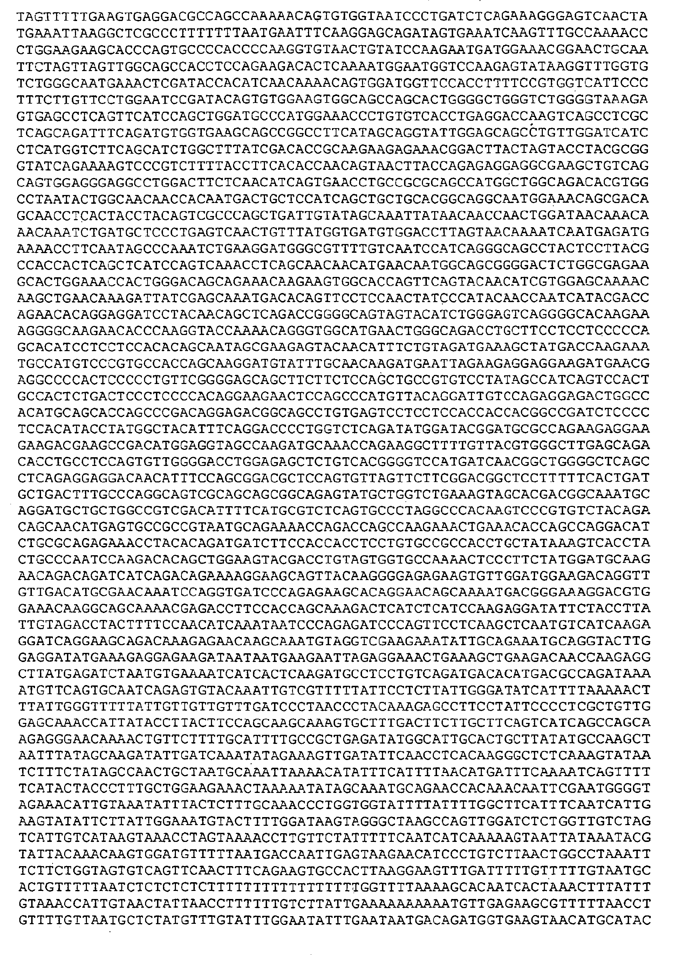

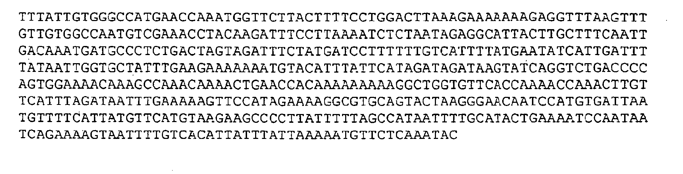

Abbildung 4 zeigt die Nukleinsäuresequenzen der 30 Markergene

### Patienten und Tumor Charakterisierung

Das Patientenkollektiv für die Entwicklung der Signatur bestand aus 55 Patienten, 34 Männer und 21 Frauen, bei denen ein kolorektales Karzinom diagnostiziert wurde. Diese Patienten wurden im Zeitraum August 1988 bis Juni 1998 operiert, um das kolorektale Karzinom total zu entfernen. Das Alter der Patienten zum Zeitpunkt der Operation reichte von 33 Jahre bis 87 Jahre, das mittlere Alter betrug 63,4 Jahre. Unter den 55 entfernten Karzinomen wurden 11 als Tumore im UICC Stadium I (TNM - Klassifikation: pT1 oder pT2 und pN0 und pM0) und 44 als Tumore im UICC Stadium II klassifiziert (TNM - Klassifikation: pT3 oder pT4 und pN0 und pM0).
Die Gesamtbeobachtungszeit der Patienten, das ist die Zeit von der ersten durchgeführten Operation bis zur letzten Beobachtung eines Patienten, betrug im Mittel 11,25 Jahre, das Minimum war 6,36 Jahre, das Maximum war 16,53 Jahre. Innerhalb der ersten drei Jahre nach der Operation wurde bei 18 Patienten eine Progression der Erkrankung diagnostiziert, 37 Patienten blieben innerhalb der ersten drei Jahre nach ihrer Operation progressionsfrei.

### Beispiel 1: RNA Extraktion und Target Labeling

Die Tumoren wurden homogenisiert und die RNA wurde mittels des RNeasy Mini Kit (Qiagen, Hilden, Deutschland). isoliert und in 55µl Wasser aufgenommen. Die cRNA Präparation wurde wie bereits beschrieben (Birkenkamp-Demtroder K, Christensen LL, Olesen SH, et al. Gene expression in colorectal cancer. Cancer Res 2002; 62:4352-63) durchgeführt. Doppelsträngige cDNA wurde mittels eines OligodT-T7 primer (Eurogentec, Koeln, Germany) synthetisiert und anschließend mit Hilfe des Promega RiboMax T7-kit (Promega, Madison,Wisconsin) und Biotin-NTP Markierungsmix (Loxo, Dossenheim, Germany) transkribiert.
Fünfzehn Mikrogramm cRNA wurden anschließend bei 95°C 35 Minuten lang fragmentiert.

### Beispiel 2: Mikroarray Experimente

Die cRNA wurde mit dem B2-Kontrollolioonukleotid (Affymetrix, Santa Clara, CA), eukaryotischen Hybridisierungskontrollen (Affymetrix, Santa Clara, CA), Heringssperm (Promega, Madison, Wisconsin), Hybridisierungspuffer and BSA auf ein finales Volumen von 300µl aufgefüllt und auf einen Mikroarraychip U133A (Affymetrix, Santa Clara, CA) 16 Stunden lang bei 45°C hybridisiert. Die Wasch- and Inkubationsschritte mit Streptavidin (Roche, Mannheim), biotinyliertem Ziege-Anti-Streptavidin Antikörper (Serva, Heidelberg), Ziege-IgG (Sigma, Taufkirchen) and Streptavidin-Phycoerythrin Konjugat (Molecular Probes, Leiden, Niederlande) wurde in einer Affymetrix Fluidics Station entsprechend dem Herstellerprotokoll durchgeführt.
Die Arrays wurden anschließend mit einem auf HP-Argon-Ionenlaser basierten konfokalen Mikroskop gescannt und die digitalisierten Bilddaten wurden mit Hilfe der Affymetrix ® Microarray Suite 5.0 Software prozessiert. Die Genechips wurden dabei einer Qualitätskontrolle unterzogen um Scans mit abnormalen Charakteristika zu entfernen. Die Kriterien waren: zu hoher oder zu niedriger dynamischer Bereich, hohe Sättigung der "perfect matches", hohes Pixelrauschen, "grid misalignment" Probleme und ein niedriges mittleres Signal-zu-Rauschen Verhältnis.

### Beispiel: 3 Bioinformatische Auswertung

Die statistische Datenanalyse wurde mit der Open-Source Software R, Version 2.3 und den Bioconductor Paketen, Version 1.8 ausgeführt. Ausgehend von den 55 CEL-Files, die Ausgabe der oben genannten Affymetrix Software sind, wurden die Gen-Expressionswerte durch FARMS Kondensierung [Hochreiter et al. (2006), Bioinformatics 22(8):943-9] bestimmt.
Basierend auf den klinischen Daten von 55 Patienten wurde das Klassifikationsproblem "Klassifikation der 55 Expressionsdatensätze nach progressionsfreiem Überleben der zugehörigen Patienten über 3 Jahre" formuliert und analysiert. Das Expressionsdatenset stammte von den oben beschriebenen Patienten, von denen bei 18 innerhalb von drei Jahren eine Progression auftrat und bei 37 progressionsfreies Überleben für drei Jahre dokumentiert wurde. Die in der Erfindung beschriebenen Markergene wurden, wie in Abbildung 3 gezeigt, mit einem zweifach geschachteltem Bootstrap-Ansatz [Efron (1979) Bootstrap Methods - Another Look at the Jackknifing, Ann. Statist. 7, 1-6], bestimmt. In der äußeren Schleife mit 500 Iterationen wurden die Daten zufällig partitioniert in ein Testset aus zwei Datensätzen und einem Trainingsset aus 53 Datensätzen. Anhand der Trainingsdaten wurde in der inneren Bootstrap-Schleife durch eine Decision-Tree-Analyse die Featurerelevanz aus den Daten extrahiert. Dazu wurden in 50 inneren Schleifeniterationen jeweils 10 Datensätze als inneres Trainingset zufällig gezogen. Diese wurden durch eine SVM, die auf den 43 verbliebenen Datensätzen trainiert wurde klassifiziert und der Einfluss eines Features aus dessen Beitrag zum Klassifikationsfehler ermittelt: Stieg der Fehler durch Permutieren der Werte eines Features auf den 10 Testdatensätzen, während die Werte aller anderen Features unverändert blieben, so wurde dieses Feature stärker gewichtet. Anhand einer Häufigkeitstabelle wurden die 30 in den inneren Schleifeniterationen am häufigsten gewählten Features ermittelt und für die Prognose der zwei Testdatensätze der äußeren Schleife verwendet: eine Support-Vector-Machine mit linearem Kernel (Kostenparameter = 10) wurde auf den 53 Datensätzen des äußeren Trainingssets trainiert und dann auf die zwei Testdatensätze angewandt. Nach 500 Iterationen wurden die durchschnittliche, prospektive Klassifikationsrate (mit Sensitivität und Spezifität) und die Häufigkeiten der gefundenen Features bestimmt.
Die Gensignaturen beinhalten nur Features, die mit großer Häufigkeit in allen Ziehungen relevant waren und wurden entsprechend der relativen Häufigkeit sortiert. In der retrospektiven "Leave-One-Out-Cross Validation" (LOOCV) der Signaturen wurden im Fall von sieben verwendeten Features 80% der Datensätze richtig klassifiziert. (siehe auch Tabelle 2)

**Tabelle 1: Markergene die eine Vorhersage zwischen progressionsfreiem Überleben und Progression der Erkrankung innerhalb der ersten drei Jahre nach der Entfernung des primären kolorektalen Karzinoms (PFS3) ermöglichen. Die Frequenz stellt die Häufigkeit dar mit der dieses Feature in den Schleifen des inneren Bootstraps einen großen Beitrag zum Klassifikationsergebnis lieferte (siehe auch Beschreibung im Beispiel 3).**

| SEQ_ID | Affymetrix_ID | HUGO_ID | RefSeq Nummer | Frequenz |
|---|---|---|---|---|
| 1 | 218360_at | RAB22A | NM_020673 | 0,8442 |
| 2 | 217286_s_at | NDRG3 | NM_022477 | 0,5014 |
| 3 | 218011_at | UBL5 | NM_024292 | 0,4966 |
| 4 | 218339_at | MRPL22 | NM_014180 | 0,4798 |
| 5 | 212671_s_at | HLA-DQA1 | NM_002122 | 0,417 |
| 6 | 203571_s_at | C10orf116 | NM_006829 | 0,4138 |
| 7 | 212958_x_at | PAM | NM_000919 | 0,3844 |
| 8 | 220639_at | TM4SF20 | NM_024795 | 0,374 |
| 9 | 211991_s_at | HLA-DPA1 | NM_033554 | 0,3644 |
| 10 | 202336_s_at | PAM | NM_000919 | 0,3594 |
| 11 | 202381_at | ADAM9 | NM_003816 | 0,346 |
| 12 | 213405_at | RAB22A | NM_020673 | 0,328 |
| 13 | 206932_at | CH25H | NM_003956 | 0,3242 |
| 14 | 218320_s_at | NDUFB11 | NM_019056 | 0,3062 |
| 15 | 204559_s_at | LSM7 | NM_016199 | 0,2924 |
| 16 | 205176_s_at | ITGB3BP | NM_014288 | 0,2706 |
| 17 | 201406_at | RPL36A | NM_021029 | 0,2648 |
| 18 | 203316_s_at | SNRPE | NM_003094 | 0,2596 |
| 19 | 209312_x_at | HLA-DRB1 | NM_002124 | 0,2548 |
| 20 | 209823_x_at | HLA-DQB1 | NM_002123 | 0,2446 |
| 21 | 218594_at | HEATR1 | NM_018072 | 0,2426 |
| 22 | 204439_at | IFI44L | NM_006820 | 0,2326 |
| 23 | 219718_at | FLJ10986 | NM_018291 | 0,2222 |
| 24 | 204670_x_at | HLA-DRB5 | NM_002125 | 0,2136 |
| 25 | 2012811_at | ADRM1 | NM_007002 | 0,2134 |
| 26 | 210312_s_at | IFT20 | NM_174887 | 0,1984 |
| 27 | 209616_s_at | CES1 | NM_001025194 | 0,1832 |
| 28 | 219640_at | CLDN15 | NM_014343 | 0,1674 |
| 29 | 201032_at | BLCAP | NM_006698 | 0,1624 |
| 30 | 213194_at | ROBO1 | NM_002941 | 0,1606 |

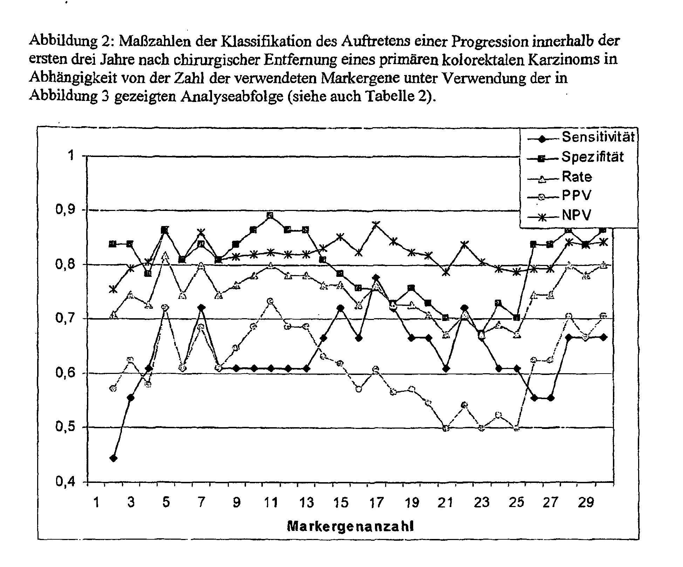

**Tabelle 2: Sensitivität, Spezifität und korrekte Klassifikationsrate für die Vorhersage des dreijährigen progressionsfreien Überlebens eines primären kolorektalen Karzinoms in Abhängigkeit von der Anzahl der verwendeten Features für das ausgewählte Patientenkollektiv (insgesamt 55 davon 18 mit Progression innerhalb von drei Jahren). Die Menge der verwendeten Gene ist monoton wachsend, das heißt in Zeile 7 sind alle Gene vc SEQ_ID 1 bis SEQ_ID 7 und in Zeile 5 alle Gene von SEQ_ID 1 bis SEQ_ID 5 zur Vorhersage herangezogen worden (siehe auch Abbildung 2).**

| SEQ_ID | HUGO_ID | Sensitivität | Spezifizität | Klassifikationsrate |
|---|---|---|---|---|
| 1 | RAB22A | NA | NA | NA |
| 2 | NDRG3 | 0,44 | 0,84 | 0,71 |
| 3 | UBL5 | 0,56 | 0,84 | 0,75 |
| 4 | MRPL22 | 0,61 | 0,78 | 0,73 |
| 5 | HLA-DQA1 | 0,72 | 0,86 | 0,82 |
| 6 | C10orf116 | 0,61 | 0,81 | 0,75 |
| 7 | PAM | 0,72 | 0,84 | 0,80 |
| 8 | TM4SF20 | 0,61 | 0,81 | 0,75 |
| 9 | BLA-DPA1 | 0,61 | 0,84 | 0,76 |
| 10 | PAM | 0,61 | 0,86 | 0,78 |
| 11 | ADAM9 | 0,61 | 0,89 | 0,80 |
| 12 | RAB22A | 0,61 | 0,86 | 0,78 |
| 13 | CH25H | 0,61 | 0,86 | 0,78 |
| 14 | NDUFB 11 | 0,67 | 0,81 | 0,76 |
| 15 | LSM7 | 0,72 | 0,78 | 0,76 |
| 16 | ITGB3BP | 0,67 | 0,76 | 0,73 |
| 17 | RPL36A | 0,78 | 0,76 | 0,76 |
| 18 | SNRPE | 0,72 | 0,73 | 0,73 |
| 19 | HLA-DRB1 | 0,67 | 0,76 | 0,73 |
| 20 | HLA-DQB1 | 0,67 | 0,73 | 0,71 |
| 21 | HEATR1 | 0,61 | 0,70 | 0,67 |
| 22 | IFI44L | 0,72 | 0,70 | 0,71 |
| 23 | FLJ10986 | 0,67 | 0,68 | 0,67 |
| 24 | HLA-DRB5 | 0,61 | 0,73 | 0,69 |
| 25 | ADRM1 | 0,61 | 0,70 | 0,67 |
| 26 | IFT20 | 0,56 | 0,84 | 0,75 |
| 27 | CES1 | 0,56 | 0,84 | 0,75 |
| 28 | CLDN15 | 0,67 | 0,86 | 0,80 |
| 29 | BLCAP | 0,67 | 0,84 | 0,78 |
| 30 | ROBO1 | 0,67 | 0,86 | 0,80 |

## Patentansprüche

1. Verfahren zur Vorhersage der Wahrscheinlichkeit des Auftretens von Rezidiven eines kolorektalen Karzinoms oder von Metastasen in entfernten Organen innerhalb der ersten drei Jahre in Patienten, deren primärer Tumor chirurgisch entfernt wurde, umfassend die Bestimmung eines Genexpressionsprofils, welches das Markergen gemäß SEQ ID NO: 1 oder ein Markergen umfasst, das mindestens 90 % Identität zu dem in SEQ ID NO: 1 dargestellten Markergen hat.

2. Verfahren nach Anspruch 1, bei dem das Expressionsprofil des Markergens mit einem Referenzmuster verglichen wird, welches indikativ für ein Wiederauftreten des kolorektalen Karzinoms eines Patienten ist.

3. Verfahren nach Anspruch 2, wobei der Vergleich der Expressionsprofile mit einem Verfahren zur Mustererkennung durchgeführt wird.

4. Verfahren nach Anspruch 3, bei dem die Mustererkennungsmethode aus einem zweifach geschachtelten Bootstrap-Ansatz in Kombination mit einer Decision-Tree-Analyse für Bestimmung der Einzelrelevanz der Gene besteht.

5. Verfahren nach einem der Ansprüche 1 bis 4, bei dem ein primäres kolorektales Karzinom untersucht wird.

6. Verfahren nach einem der Ansprüche 1 bis 5, bei dem ein primäres kolorektales Karzinom der Stadien UICC-I oder UICC-II untersucht wird.

7. Verfahren nach einem der Ansprüche 1 bis 6, bei dem das Expressionsprofil einer minimal zwei Gene enthaltenen Kombinationen aus den 28 Markergenen der Tabelle 1 bestimmt wird.

8. Verfahren nach einem der Ansprüche 1 bis 7, wobei das Expressionsprofil des Markergens aus einer Probe des Patienten gewonnen wird.

9. Verfahren nach Anspruch 8, wobei das Expressionsprofil der Markergene durch die Messung der Quantität der Markergen-mRNA bestimmt wird, insbesondere mittels Genchiptechnologie, (RT-) PCR, Northern Hybridisierung, Dot-Blotting oder in situ Hybridisierung.

10. Verwendung einer Nukleinsäure der Nukleinsäuresequenz gemäß SEQ ID NO: 1 zur Vorhersage der Wahrscheinlichkeit des Auftretens von Rezidiven oder Metastasen in entfernten Organen eines Patienten mit kolorektalem Karzinom oder eines Markergens, das mindestens 90 % Identität zu dem in SEQ ID NO: 1 dargestellten Markergene hat, ihren revers-komplementären Sequenzen oder Kombinationen daraus, welche geeignet sind, die differentielle Expression der Sequenzen zu detektieren.

11. Verwendung eines cDNA- oder Oligonukleotid-Mikroarrays welcher eine Nukleinsäuresequenz gemäß SEQ ID NO: 1 enthält in einem Verfahren nach den Ansprüchen 1 bis 9.

12. Verwendung eines Kits in einem Verfahren nach den Ansprüchen 1 bis 9, welcher Material zur Detektion einer Nukleinsäuresequenzen gemäß SEQ ID NO: 1 enthält.

13. Verwendung eines Kits gemäß Anspruch 12, welcher ein Mikroarray gemäß Anspruch 11 enthält.

## Claims

1. A method for the prediction of the likelihood of occurrence of recrudescence relapse of a colorectal carcinoma or of metastases in remote organs within the first three years in patients whose primary tumor has been surgically removed, comprising the determination of a gene expression profile comprising a marker gene according to SEQ ID NO: 1 or a marker gene with at least 90% identity to the marker gene depicted in SEQ ID NO: 1.

2. The method of claim 1, wherein the expression profile of the marker gene is compared to a reference pattern which is indicative of a recurrence of colorectal carcinoma in a patient.

3. The method of claim 2, wherein the comparison of the expression profiles is performed with a method for pattern recognition.

4. The method of claim 3, wherein the pattern recognition method consists of a doubly-nested bootstrap approach in combination with a Decision-Tree analyses for determining the individual relevance of the genes.

5. The method according to one of claims 1 to 4, wherein a primary colorectal carcinoma is examined.

6. The method according to one of claims 1 to 5, wherein a primary colorectal carcinoma of stages UICC-I or UCICC-II is examined.

7. The method according to one of claims 1 to 6, wherein the expression profile of a combination comprising at least two genes from the 28 marker genes of table 1 is determined.

8. The method of one of claims 1 to 7, wherein the expression profile of the marker genes is obtained from a patient sample.

9. The method of claim 8, wherein the expression profile of the marker genes is determined through the measurement of the quantity of the marker gene mRNA, in particular using gene chip technology, (RT-) PCR, Northern hybridization, dot-blotting or in situ hybridization.

10. Use of a nucleic acid of the nucleic acid sequence according to SEQ ID NO: 1 for the prediction of the likelihood of the occurrence of relapse or metastasis in remote organs of a patient with colorectal carcinoma or of a marker gene with at least 90 % identity to the marker gene depicted in SEQ ID NO: 1, its reverse complementary sequences or combinations thereof, which are suitable for detecting the differential expression of the sequences.

11. Use of a cDNA- or oligonucleotide-microarray comprising a nucleic acid sequence according to SEQ ID NO: 1 in a method of claims 1 to 9.

12. Use of a kit a method according to claims 1 to 9 comprising material for the detection of a nucleic acid sequence according to SEQ ID NO: 1.

13. Use of a kit of claim 12, comprising a microarray according to claim 11.

## Revendications

1. Procédé pour la prédiction de la probabilité de l'apparition de récidive d'un carcinome colorectal ou de métastases dans des organes éloignés dans les trois premières années chez des patients dont la tumeur primaire a été éliminée par voie chirurgicale, comprenant la détermination d'un profil d'expression génique, qui comprend le gène marqueur selon SEQ ID NO: 1 ou un gène marqueur qui a une identité d'au moins 90 % avec le gène marqueur représenté dans SEQ ID NO: 1.

2. Procédé selon la revendication 1, dans lequel le profil d'expression du gène marqueur est comparé à un modèle de référence, qui est indicatif d'une récurrence du carcinome colorectal d'un patient.

3. Procédé selon la revendication 2, dans lequel la comparaison des profils d'expression est réalisée par un procédé de reconnaissance de modèle.

4. Procédé selon la revendication 3, dans lequel la méthode de reconnaissance de modèle est constituée d'une approche bootstrap à double emboîtement en combinaison avec une analyse par arbre de décision pour la détermination de la pertinence individuelle des gènes.

5. Procédé selon l'une des revendications 1 à 4, dans lequel on examine un carcinome colorectal primaire.

6. Procédé selon l'une des revendications 1 à 5, dans lequel on examine un carcinome colorectal primaire aux stades UICC-1 ou UICC-2.

7. Procédé selon l'une des revendications 1 à 6, dans lequel on détermine le profil d'expression d'une combination, contenant au minimum deux gènes, des 28 gènes marqueurs du Tableau 1.

8. Procédé selon l'une des revendications 1 à 7, dans lequel on obtient le profil d'expression du gène marqueur à partir d'un échantillon du patient.

9. Procédé selon la revendication 8, dans lequel on détermine le profil d'expression des gènes marqueurs par mesure de la quantité de l'ARN des gènes marqueurs, en particulier par la technologie des puces à ADN, la (RT)-PCR, l'hybridation de Northem, le dot-blotting ou l'hybridation in situ.

10. Utilisation d'un acide nucléique ayant la séquence d'acide nucléique selon SEQ ID NO: 1 pour prédire la probabilité de l'apparition de récidives ou de métastases dans des organes éloignés d'un patient ayant un carcinome colorectal, ou d'un gène marqueur qui a une identité d'au moins 90 % avec les gènes marqueurs représentés dans SEQ ID NO: 1, leurs séquences complémentaires inverses ou leurs combinaisons, qui sont aptes à détecter une expression différentielle des séquences.

11. Utilisation d'un micro-réseau d'ADNc ou d'oligonucléotides, qui contient une séquence d'acide nucléique selon SEQ ID NO: 1, dans un procédé selon les revendications 1 à 9.

12. Utilisation d'une trousse dans un procédé selon les revendications 1 à 9, qui contient un matériel pour la détection d'une séquence d'acide nucléique selon SEQ ID NO: 1.

13. Utilisation d'une trousse selon la revendication 12, qui contient un micro-réseau selon la revendication 11.
